# EUROPEAN PATENT SPECIFICATION

(11) **EP 2 580 242 B1**
(45) Date of publication and mention of the grant of the patent: **06.04.2016**
(21) Application number: 11725865.7
(22) Date of filing: 14.06.2011
(51) Int. Cl.: C07K 16/28, A61K 38/18, A61K 38/00

(54) **MODULATION OF THE INTERACTION BETWEEN SORLA AND GDNF-FAMILY LIGAND RECEPTORS**
MODULATION DER INTERAKTION ZWISCHEN LIGANDREZEPTOREN DER SORLA- UND GDNF-FAMILIE
MODULATION DE L'INTERACTION ENTRE SORLA ET LES RÉCEPTEURS DES LIGANDS DE LA FAMILLE DU GDNF

(30) Priority: 14.06.2010 US 354536 P; 14.06.2010 DK 201000522
(43) Date of publication of application: 17.04.2013
(73) Proprietor: H. Lundbeck A/S, 2500 Valby (DK)
(72) Inventor: NYKJÆR, Anders, DK-8240 Risskov (DK); GLERUP, Simon, DK-8240 Risskov (DK); MADSEN, Peder Søndergaard, DK-8240 Risskov (DK); PETERSEN, Claus Munck, DK-8000 Århus C (DK); OLSEN, Ditte, DK-8210 Århus V (DK)
(74) Representative: Damsgaard, Mads
(86) International application number: PCT/DK2011/050214
(87) International publication number: WO 2011/157275

(56) References cited:
- WO-A2-2009/155932
- WO-A2-2010/069331
- WILLNOW T E ET AL: "VPS10P-domain receptors regulators of neuronal viability and function", NATURE REVIEWS NEUROSCIENCE, NATURE PUBLISHING GROUP, LONDON, GB, vol. 9, no. 12, 1 December 2008 (2008-12-01), pages 899-909, XP009152987, ISSN: 1471-0048, DOI: 10.1038/NRN2516

## Description

### FIELD OF THE INVENTION

The present invention relates to the modulation of the Vps10p-domain receptor SorLA and its interactions with GDNF-family ligand receptors. Agents that are able to modulate or inhibit this interaction have a potential in the treatment of mental and behavioral disorders. The invention provides such ligands capable of acting as modulators of signaling via SorLA in addition to but not limited to the retrograde transport and sorting of GDNF. The agents are thus selected from antagonists/inhibitors.

### BACKGROUND OF THE INVENTION

Diseases of the nervous system and in particular mental and behavioural disorders are among the leading causes of disability, accounting for more than 37 percent of years of life lived with disability amongst adults aged 15 years and older worldwide, and as illness likely to represent an increasingly greater health, societal and economic problem in the coming years (Lopez and Murray 1998).

Mental and behavioral disorders are defined in e.g. ICD10, Chapter V, Blocks F00-F99 (Mental and behavioral disorders) from World Health Organization, and includes for example major depressive disorders, schizophrenia, attention deficit and hyperactivity disorder (ADHD), drug abuse, anxiety disorders, and bipolar disorder (manic depressive illness).

These disorders are common, severe, chronic, and often life-threatening illness. Suicide is estimated to be the cause of the death in up to 15% of the individuals with disorders such as major depressive disorders and bipolar disorder, and many other deleterious health-related effects have been recognized (Michelson, Stratakis et al. 1996; Musselman, Evans et al. 1998; Ciechanowski, Katon et al. 2000; Schulz, Beach et al. 2000; Kupfer 2005). It is increasingly being recognized that some of these disorders are systemic diseases with deleterious effects on multiple organ systems.

Altered neuronal activity, survival, and in particular impairment in synaptic plasticity and transmission is believed to underlie the pathophysiology of neuronal disease. The neurotransmitter dopamine, in particular, is involved in several common disorders of brain function, notably Parkinson's disease, schizophrenia, attention deficit and hyperactivity disorder, as well as in drug dependence and certain endocrine disorders. Many of the drugs clinically used to treat these conditions work by influencing dopamine transmission. There are three main dopaminergic pathways. The nigrostriatal pathway that is important in motor control and the selective degeneration of the dopaminergic neurons of this pathway is a hall-mark of Parkinson's disease. The mesolimbic/mesocortical pathways are running from groups of cells in the midbrain to parts of the limbic system, especially the nucleus accumbens, and to the cortex; they are involved in emotion and drug-induced reward systems. Finally, the tuberohypophyseal pathway running from the hypothalamus to the pituitary gland, the secretions of which they regulate.

Neurotrophic factors are potent mediators of neuronal "stay alive" signals and have profound effect on synaptic transmission and plasticity. Glia cell-derived neurotrophic factor (GDNF) is one of a group of related homodimeric neurotrophic factors that also includes neuturin (NRTN), persephin (PSPN) and artemin (ARTN). Together, these are denoted GDNF-family ligands (GFL). GDNF is expressed throughout the developing nervous system, but in the adult brain it is particularly highly expressed in neurons of striatum, thalamus, cortex and hippocampus. Due to retrograde transport, a significant amount of GDNF, originating from striatum, is furthermore found in the substantia nigra. Expression is also explicit astrocytes and Schwann cells as well as in non-neuronal tissues like kidney, testis, and skeletal muscle.

### SorLA

Sorting protein-related receptor abbreviated SorLA (Swiss prot ID no Q92673), also known as LR11, is a 250-kDa type-1 membrane protein and the second member identified in the Vps10p-domain receptor family. All the receptors in this family share the structural feature of an approximately 600-amino acid N-terminal the luminal portion of the yeast sorting receptor Vps10p (Marcusson, Horazdovsky et al. 1994). The Vps10p-domain (Vps10p-D) that among other ligands binds neurotrophic factors and neuropeptides (Mazella, Zsurger et al. 1998; Munck Petersen, Nielsen et al. 1999; Nykjaer, Lee et al. 2004; Westergaard, Sorensen et al. 2004; Teng, Teng et al. 2005), constitutes the entire luminal part of the first identified member Sortilin and is activated for ligand binding by enzymatic propeptide cleavage (Mazella, Zsurger et al. 1998; Munck Petersen, Nielsen et al. 1999). SorLA, like sortilin, whose lumenal domain consists of a Vps10p domain only, is synthesized as a proreceptor that is cleaved by furin in late Golgi compartments. It has been demonstrated that propeptide cleavage conditions the Vps10p domain for propeptide inhibitable binding of neuropeptides and the receptor-associated protein. The sequence of the SorLA vps10-p domaine is given in SEQ ID NO 3. It has been demonstrated (Jacobsen, Madsen et al. 2001) that avid binding of the receptor-associated protein, apolipoprotein E, and lipoprotein lipase not inhibited by propeptide occurs to sites located in other lumenal domains. In transfected cells, about 10% of full length SorLA is expressed on the cell surface capable mediating endocytosis. The major pool of receptors is found in late Golgi compartments, and interaction with newly synthesized ligands has been suggested. SorLA is highly expressed in distinct cell types throughout the nervous system both during development and in the adult organism (Kanaki, Bujo et al. 1998; Motoi, Aizawa et al. 1999; Offe, Dodson et al. 2006). Interestingly, SorLA levels are reduced in the sporadic form of Alzheimer's disease (Scherzer, Offe et al. 2004; Dodson, Gearing et al. 2006; Sager, Wuu et al. 2007) and inherited mutations in the SorLA gene are genetically linked to late-onset Alzheimer's disease (Rogaeva, Meng et al. 2007). Importantly, SorLA has been shown to mediate high affinity binding and sorting of amyloid precursor protein, and to confer protection against Aβ generation (Andersen, Reiche et al. 2005; Offe, Dodson et al. 2006; Spoelgen, von Arnim et al. 2006; Rogaeva, Meng et al. 2007).

WO 2009/155932 discloses an antagonistic antibody directed against the extracellular domain of SorLA for the treatment of pain. WO 2004/056385 discloses antibodies directed against e.g. the extracellular domain of SorLA for the possible treatment of neurological diseases e.g. Parkinson's disease and Alzheimer's disease.

Although a number of drugs are already available for the treatment of neurological, mental and behavioral disorders, all have complex indirect mechanism of action, and are aimed at alleviating the symptoms rather than at treating the underlying cause of the disease. Instead, it is generally believed that drugs that target the signaling pathways that regulate synaptic plasticity or neuronal survival should be developed as long-term treatments for neurological, mental and behavioural disorders (Manji, Drevets et al. 2001). Extensive experimental and clinical data suggest a central function for GDNF signaling in mental and behavioral disorders, and in disorders of the nervous system in general. For example, polymorphisms in the GDNF or GFRα1-3 genes correlate with schizophrenia, and GDNF polymorphisms are associated with attention deficit and hyperactivity disorder.

### SUMMARY OF THE INVENTION

The present invention relates to a method to increase the survival of neurons, such as dopaminergic neurons, by modulating the interaction between SorLA and GDNF-family ligand receptors, such as the GFRα1, 2, 3, and/or 4 receptors. According to one embodiment the extracellular levels of GDNF in the brain of a patient in the need thereof may be increased by modulating or inhibiting the interaction between SorLA and GFRα1, or the internalisation and/or the degradation of GDNF is inhibited.

In particular the present invention relates to a method, wherein the agent used to modulate the interaction between the SorLA and GDNF-family ligand receptors is selected from proteins, peptides, antibodies or small organic compounds.

The invention also relates to a pharmaceutical composition comprising such agents in combination with one or more pharmaceutically acceptable carriers or diluents or in combination with an adjuvant.

Additionally the invention relates to the use of such agents or pharmaceutical compositions to increase the survival of neurons, such as e.g. dopaminergic neurons, in particular within the diseases comprising attention deficit and hyperactivity disorder (ADHD), drug abuse and axiety disorder.

### BRIEF DESCRIPTION OF DRAWINGS

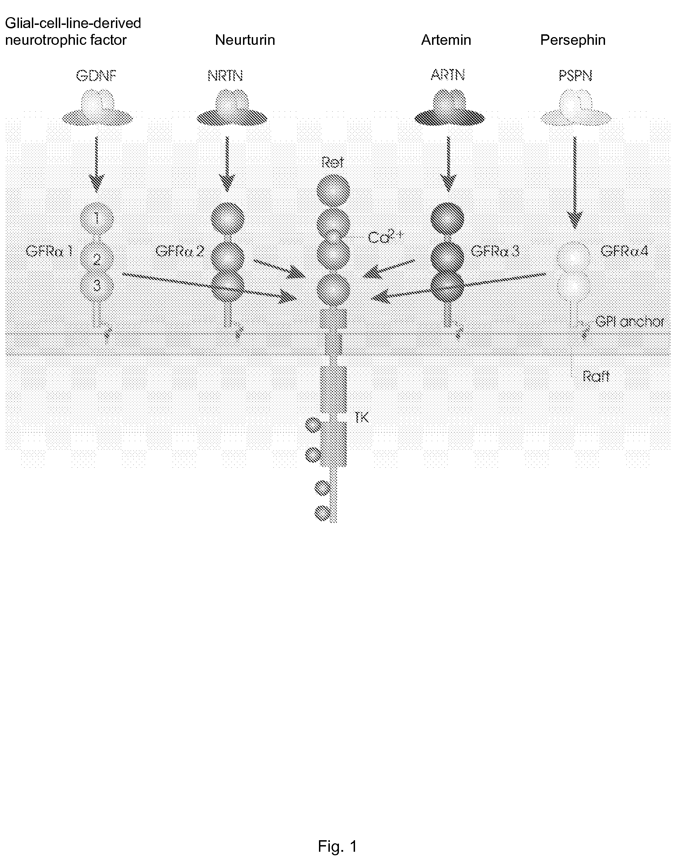

Figure 1. GDNF family ligand signaling

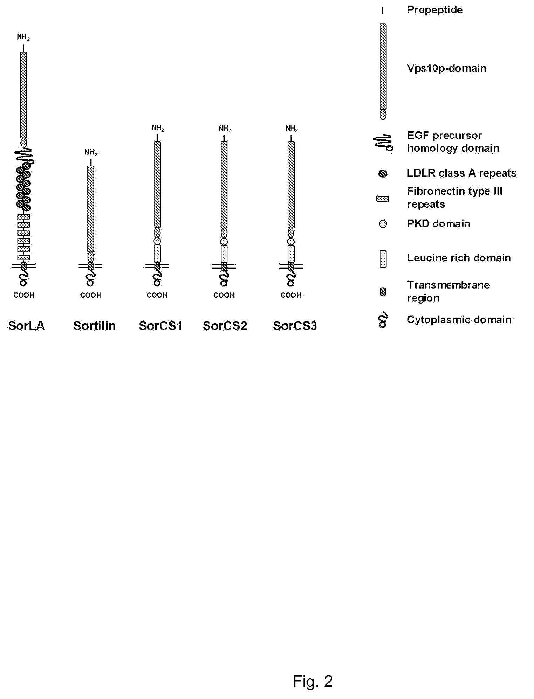

Figure 2. Domain structure of Vps10p-domain receptors

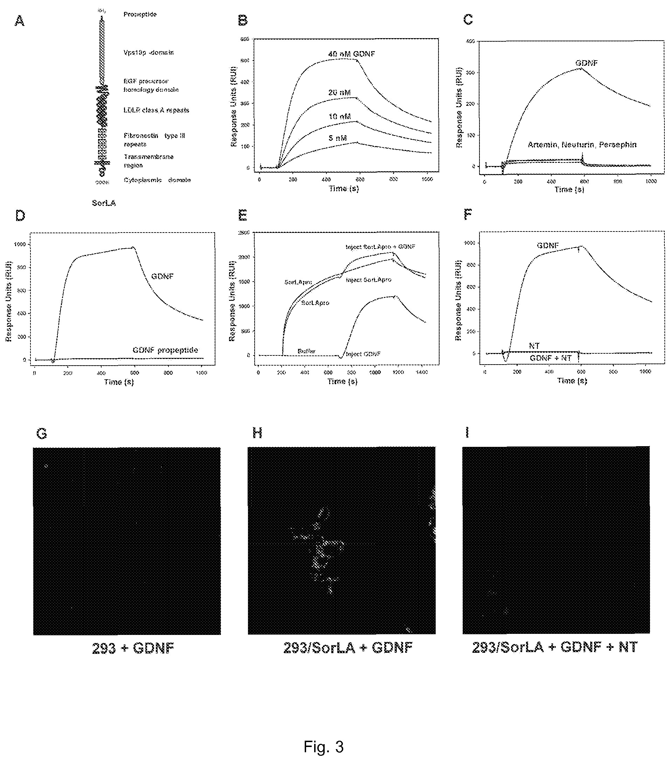

Figure 3. SorLA selectively binds GDNF but not other GDNF-family ligands (A) The domain structure of SorLA is depicted. (B) GDNF binds to immobilized SorLA in a concentration-dependent manner as shown by SPR. The calculated KD is in the range of 3-8 nM. (C) SorLA binding is selective for GDNF and not the other GDNF family ligands, artemin, neuturin, and persephin. The neurotrophic factor concentrations used are 20 nM. (D) The interaction is mediated by the mature part of GDNF and not the GDNF propeptide (GDNFpro). Sensorgrams of 100 nM ligand concentration are shown. (E) Excess SorLA propeptide (SorLApro, 10 µM) partially inhibits GDNF binding (100 nM). (F) Binding of GDNF (50 nM) is completely inhibited by excess neurotensin (NT, 20 µM). (G-H) Internalization of 50 nM GDNF by 293 cells or 293 cells stably expressing SorLA. Internalized GDNF (green) was visualized by immunofluorescense. (I) GDNF internalization was inhibited by excess neurotensin (NT, 20 µM).

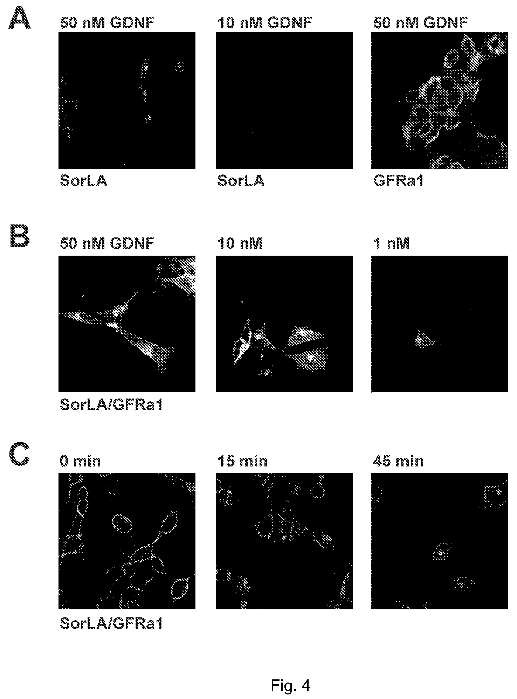

Figure 4. Cooperative GDNF uptake by SorLA and GFRα1
   (A) GDNF (50 nM) internalized by SorLA during 45 min was visualized by immunofluoerescense. However, internalization could not be detected using 10 nM GDNF. GDNF (50 nM) was not internalized by cells expressing GFRα1 but remained bound to surface. (B) GDNF is internalized to a large extent by cells expressing both SorLA and GFRα1. (C) Time course of the process of GDNF (10 nM) internalization from the surface by SorLA and GFRα1.

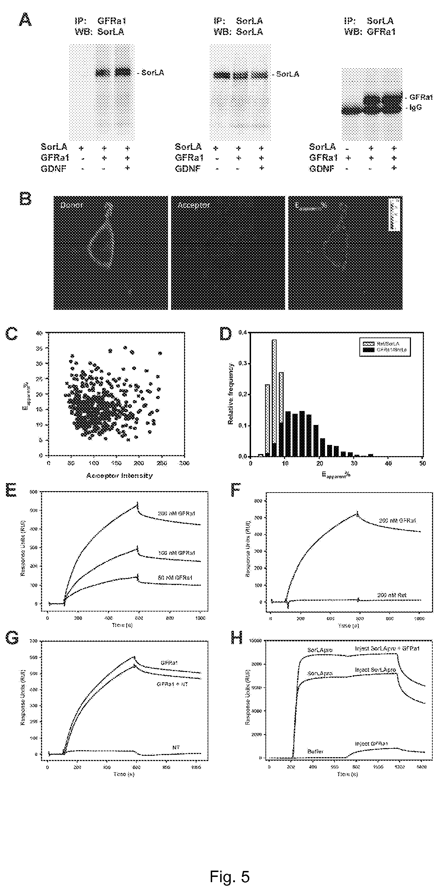

Figure 5. SorLA and GFRα1 forms a cell surface GDNF receptor complex
   (A) SorLA interacts directly with GFRα1 in cells as shown by co-immunoprecipitation of SorLA with GFRα1 from cells expressing both receptors. (B) Fluorescence resonance energy transfer (FRET) analysis of the cell surface interaction between SorLA (acceptor) and GFRα1 (donor) in non-permeabilized fixed cells using antibodies against the extracellular domains and fluorescently-labelled secondary antibodies. A representative cell is shown. (C) Cell surface SorLA/GFRα1 complex formation is highly specific. Mean Eₐₚₚ% values were calculated within regions of interest (ROI) of size 5x5 pixels in all FRET channel images and are plotted as a function of the corresponding mean acceptor signals of the ROIs. The nearly constant dependence of Eₐₚₚ% on the acceptor signal is indicative of a specific interaction. (D) Histogram showing normal distribution of Eₐₚₚ% values with a mean of 16% for the SorLA/GFRα1 interaction (black histogram). For comparison, the Eₐₚₚ% values were measured for SorLA and Ret in stably transfected cells (grey histogram). The mean Eₐₚₚ% value was 6% suggesting that a potential SorLA/Ret interaction is not specific. (E) GFRα1 binds to the immobilized SorLA extracellular domain in a concentration dependent manner with a KD of approximately 6 nM. (F) Specific interaction of GFRα1 but not Ret extracellular domain (200 nM of either) with SorLA. (G) Unlike the SorLA/GDNF interaction, GFRα1 (200 nM) binding by SorLA is not inhibited by excess neurotensin (NT, 20 µM). (H) GFRα1 (200 nM) binding to SorLA is inhibited by excess SorLA propeptide (5 µM).

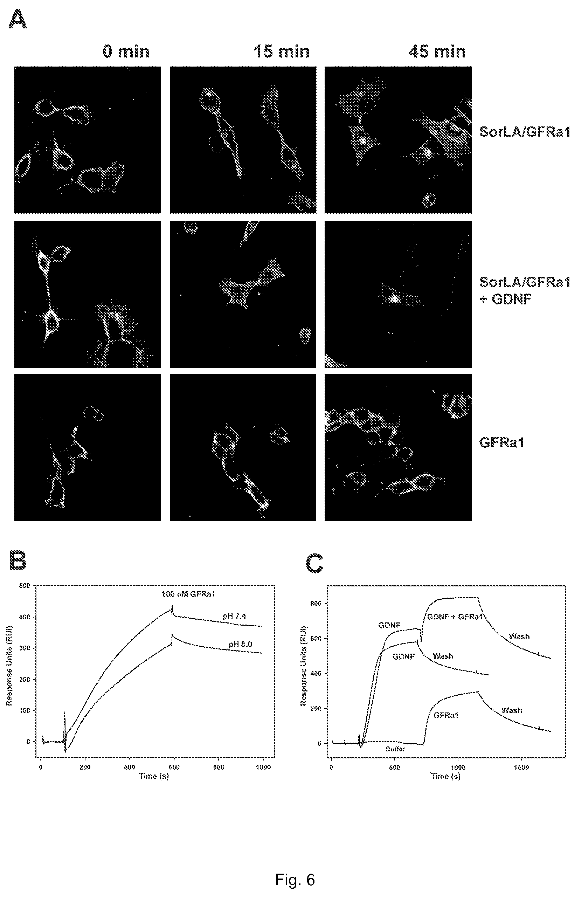

Figure 6. Retrograde sorting of GFRα1/GDNF by SorLA
   (A) GFRα1 (green) is internalized from the cell surface over time in the presence of SorLA. (B) The internalization appears to be enhanced by the presence of GDNF. (C) GFRα1 alone is not internalized during the course of the experiment. (D) The SorLA/GFRα1 interaction is stable at low pH, suggesting the complex persists at endosomal pH. (E) SorLA/GDNF and SorLA/GFRα1 are not competive interactions as shown by SPR analysis of the binding 50 nM of GDNF and 50 nM GFRα1 to immobilized SorLA.

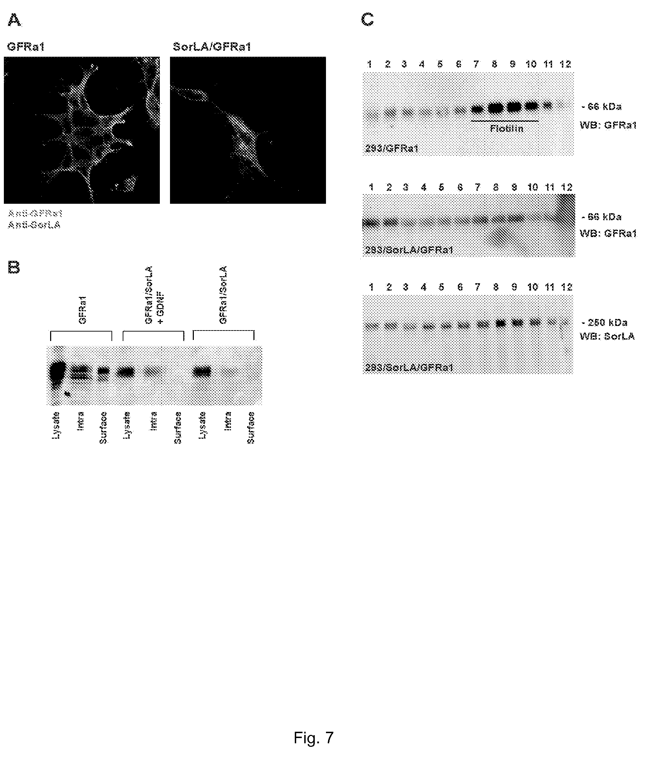

Figure 7. SorLA regulates GFRα1 subcellular localization
   (A) The presence of SorLA results in a more vesicle-like localization of GFRα1 in stably transfected cells. (B) Surface biotinylation experiments showing a reduction in surface localized GFRα1 in cells expressing SorLA. (C) SorLA decreases the relative amount of GFRα1 in fractions containing flotilin, a lipid raft marker, as assessed by sucrose gradient centrifugation.

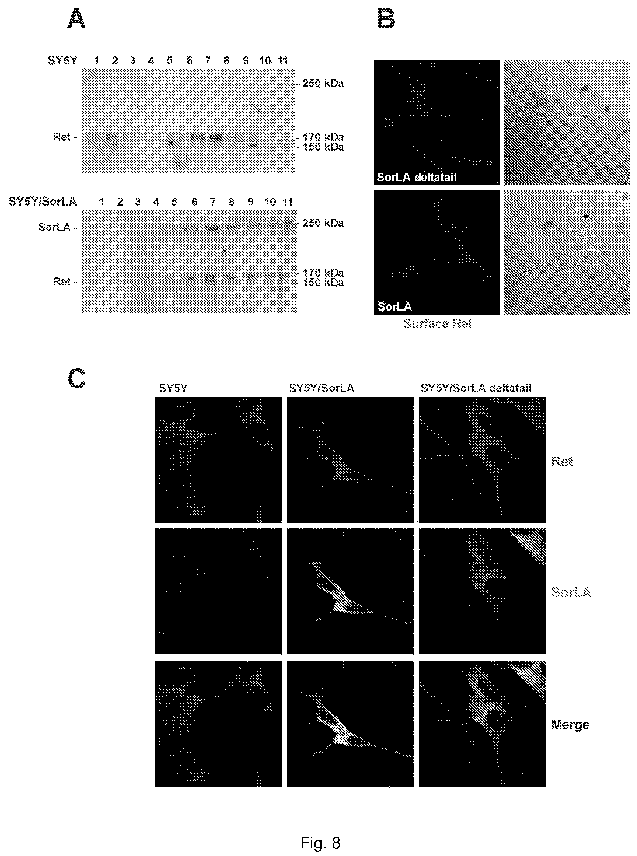

Figure 8. SorLA disrupts Ret surface clustering
   (A) Overexpression of SorLA alters the subcellular localization of endogenous Ret in SY5Y neuroblastoma cells as shown by equilibrium gradient centrifugation. (B) Ret stainings of non-permeabilized differentiated SY5Y cells showing that the presence of surface localized Ret clusters disappears when wild-type SorLA is overexpressed but not by overexpression of SorLA deltatail. (C) Immunofluorescense stainings of differentiated neuroblastoma (SY5Y) cells expressing endogenous Ret and some SorLA, overexpressing wild-type SorLA or a C-terminal truncated SorLA variant lacking the cytoplasmic tail (SorLA deltatail). In non-transfected cells, the endogenous Ret is observed in intensely stained dispersed clusters. However, Ret clustering is completely disrupted by SorLA overexpression. Truncation of the SorLA cytoplasmic tail rescues Ret clustering.

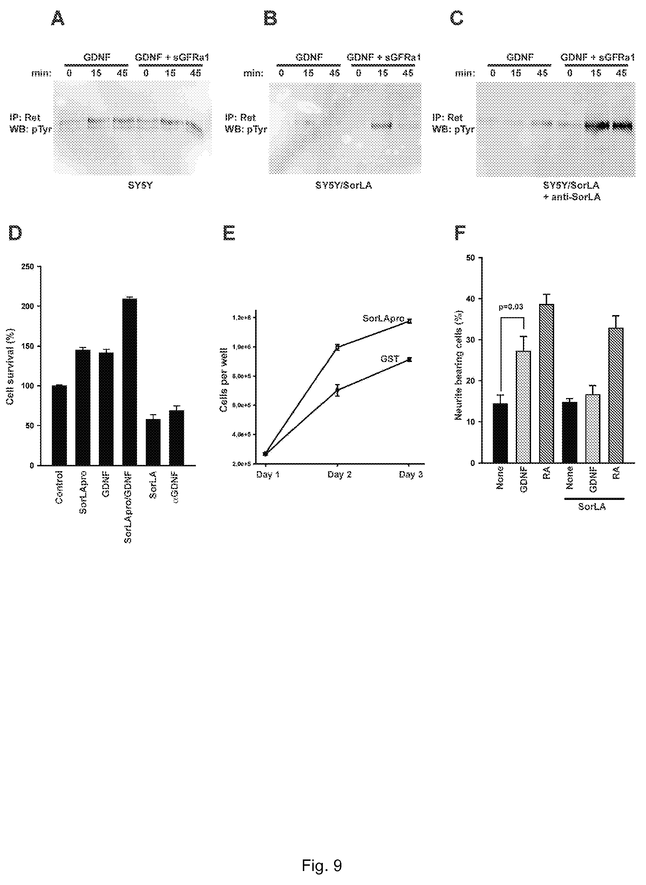

Figure 9. SorLA modulates Ret signaling and downstream functions
   (A) Stimulation of SY5Y neuroblastoma cells with 6 nM GDNF +/- 6 nM soluble GFRα1 for 0, 15, and 45 min. Ret was immunoprecipitated using anti-Ret antibodies and analyzed by Western blotting using anti-phosphotyrosine antibodies. (B) A similar experiment using neuroblastoma cells overexpressing SorLA. (C) SorLAs inhibition of Ret phosphorylation was rescued by the presence of 10 µg/ml anti-SorLA antibodies during the experiment. (D) Inhibition of endogenous SorLA by the presence of excess propeptide (*SorLApro*) increases the survival of neuroblastoma cells whereas overexpression (*SorLA*) results in reduced survival. (E) Inhibition of SorLA by excess propeptide increases proliferation of neuroblastoma cells (F) Overexpression of SorLA inhibits GDNF-induced but not retinoic acid (RA)-induced neurite outgrowth.

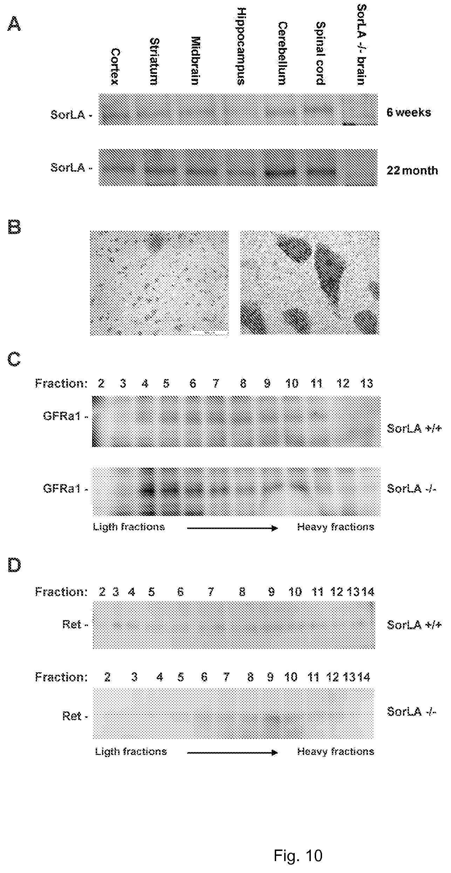

Figure 10. SorLA regulates GFRα1 and Ret subcellular localization throughout the brain
   (A) SorLA is expressed throughout the central nervous system of young and old mice shown by Western blotting. (B) Immunohistochemistry showing the presence of SorLA in vesicles surrounding the soma of neurons throughout the cortex. (C) Altered subcellular localization of GFRα1 in SorLA -/- brain assessed by sucrose gradient centrifugation of brain homogenates from wild-type or SorLA -/mice. (D) A similar experiment showing altered subcellular localization of Ret in SorLA -/- brain.

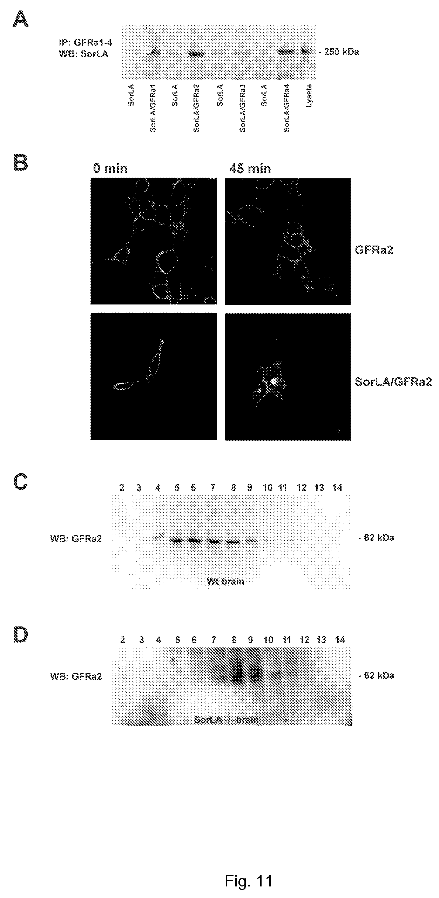

Figure 11. SorLA is a general sorting receptor for GFRas
   (A) Cells stably expressing SorLA were transiently transfected with GFRα1-4 as indicated, which was subsequently immunoprecipitated using anti-GFRα1, -2, -3, and -4, respectively, followed by Western blotting using anti-SorLA. (B) Retrograde sorting of GFRα2 (green) in the presence of SorLA. (C) Altered subcellular localization of GFRα2 in SorLA -/- brains as assessed using sucrose gradient centrifugation.

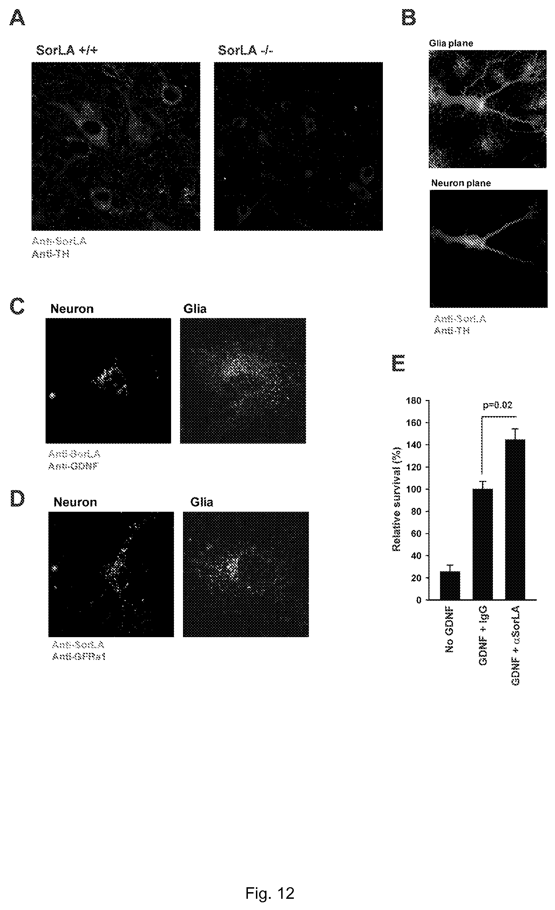

Figure 12. SorLA inhibition increases GDNF-induced survival of dopaminergic neurons
   (A) Immunohistochemistry on the substantia nigra showing the presence of SorLA (green) in dopaminergic neurons (red). (B), Primary culture of dopaminergic neurons (red) grown on a glia cell layer. SorLA is expressed in both glia and neurons. (C) Colocalization of SorLA and GDNF in neurons and glia of primary dopaminergic neurons. (D) Colocalization of SorLA and GFRα1 in neurons and glia of primary dopaminergic neurons. (E) Survival of primary dopaminergic neurons requires GDNF and is promoted by anti-SorLA antibodies.

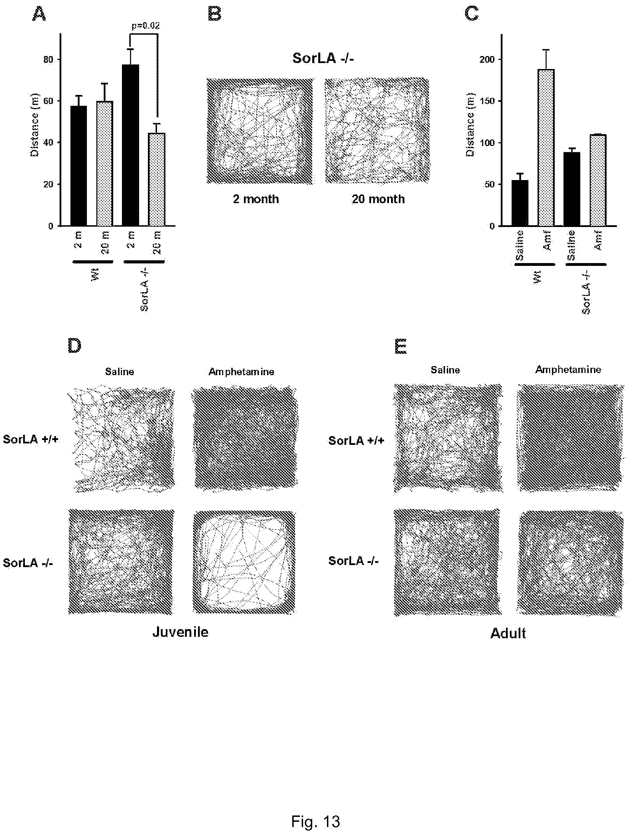

Figure 13. SorLA -/- mice display hyperactivity and insensitivity to amphetamine
   (A) SorLA -/- mice are hyperactive when tested in an open field. Hyperactivity is reversed by age. (B) Track plots of young and old SorLA -/- mice during 20 min in the open field. (C) SorLA -/- mice are insensitive to 10 mg/kg amphetamine. (D) Track plots of juvenile (less than 8 weeks old) wild-type and SorLA -/- mice during 40 min in the open field following injection of saline or amphetamine. (E) Track plots of adult (more than 12 weeks old) wild-type and SorLA -/- mice during 40 min in the open field following injection of saline or amphetamine.

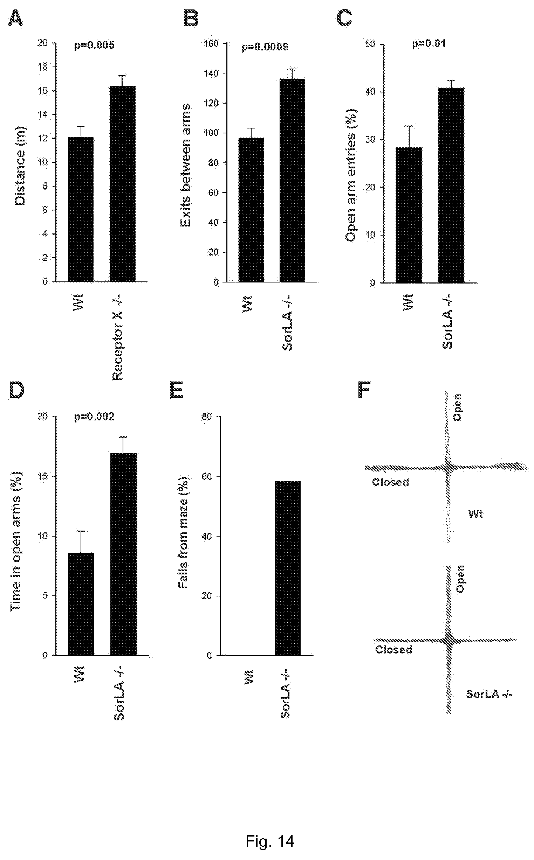

Figure 14. SorLA -/- mice show increased risk-taking behavior and attention deficits
   (A) SorLA -/- mice are hyperactive when tested in an elevated plus maze. (B) SorLA -/- mice perform increased exits between arms in an elevated plus maze. (C) SorLA -/- mice perform increased entries into open arms relative to total exits between arms. (D) SorLA -/- mice spend an increased percentage of time in the open arms. (E) Falls of the elevated plus maze for wild-type (n=23) and SorLA -/(n=12) mice. (F) Track plots of wild-type and SorLA -/- mice tested for 10 min in an elevated plus maze.

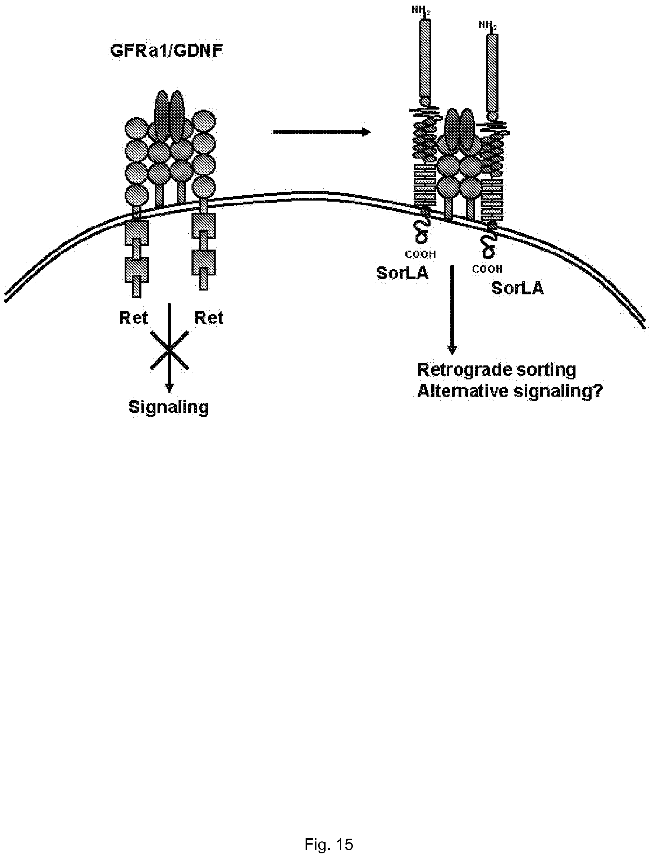

Figure 15. Hypothetical model describing retrograde sorting of GDNF receptors by SorLA

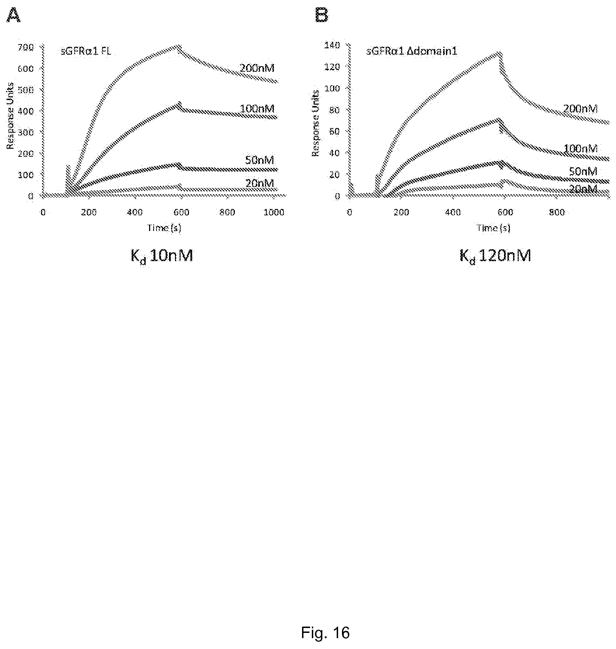

Figure 16. Truncation of GFRα1 domain 1 results in reduced affinity for SorLA.
   (A) soluble full length GFRα1 (sGF Rα1 FL) binds to SorLA in a concentration-dependent manner with an estimated Kd = 12 nM. (B) GFRα1 consists of three homologous domains. An N-terminal truncated soluble GFRα1 variant (s GFRα1 Δdomain 1) lacking a sequence stretch corresponding to domain 1 binds immobilized SorLA with a Kd= 120 nM.

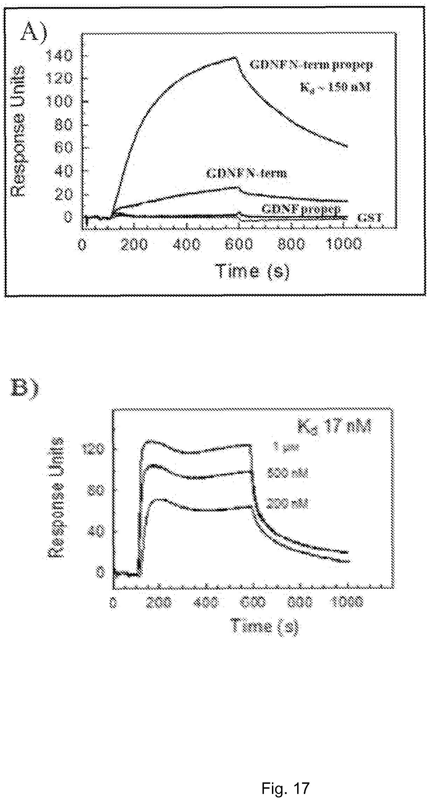

Figure 17. The N-terminal 38 amino acids of mature GDNF bind to SorLA.
   (A) fusion proteins between GST and the GDNF propeptide (GDNFpropep), the N-terminal 38 amino acids of mature GDNF (GDNFN-term), or the GDNF propeptide followed by the first 38 amino acids of mature GDNF (GDNFN-term propep) binding to immobilized SorLA. GST alone is included as negative control. (B) a peptide encompassing the first 38 amino acids of mature GDNF binds to SorLA in concentration-dependent manner.

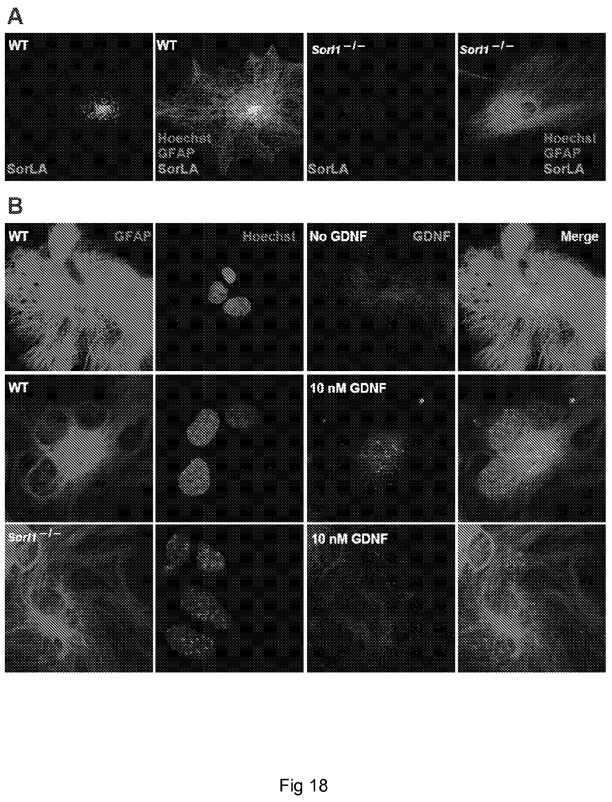

Figure 18. SorLA is a GDNF internalization receptor in the CNS
   (A) Primary cultures of cortical glia cells express high levels of SorLA. (B) GDNF (10 nM, 15 min) is internalized by glia cells from wild-type but not Sorl1-/- mice.

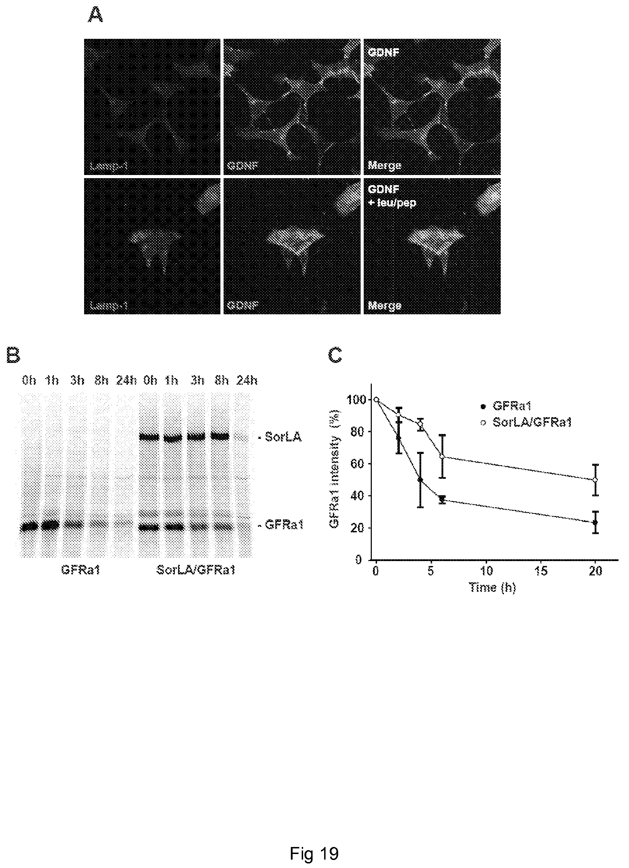

Figure 19. GDNF, not GFRα1, is sorted to lysosomes for degradation
   (A) Inhibition of lysosomal degradation increases internalized GDNF levels in cells expressing SorLA and GFRα1. (B-C) Turnover of GFRα1 in the absence or presence of SorLA as assessed by metabolic labelling followed by pulse chase analysis.

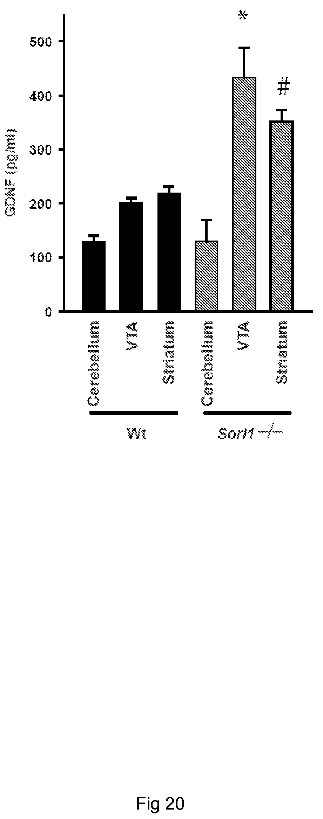

Figure 20. Increased GDNF levels in SorLA knockout mice
   GDNF levels determined by ELISA in tissues from wild-type and Sorl1 knockout mice. GDNF is increased in VTA and striatum of knockout animals (P=0.01, n=3; each comprising a pool of three animals).

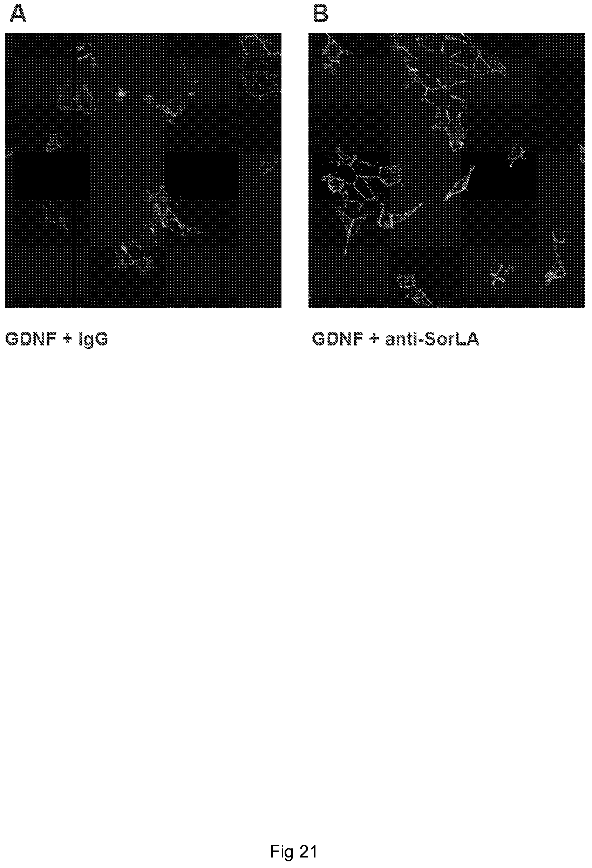

Figure 21. Inhibition of GDNF internalization and degradation by SorLA function blocking antibodies. (A) 293 cells expressing GFRa1 and SorLA were preincubated with non-specific rabbit IgG (10 ug/ml) for 2h and subsequently with both GDNF (10 nM) and rabbit IgG for 15 min. Cells were then fixed and stained using GDNF antibodies and fluorescently labelled secondary antibodies. (B) a similar experiment using rabbit polyclonal antibodies raised against the extracellular domain of SorLA. The presence of anti-SorLA IgG increases surface GDNF immunofluorescence.

### DETAILED DESCRIPTION OF THE INVENTION

### Definitions

Adjuvant: Any substance whose admixture with an administered immunogenic determinant / antigen increases or otherwise modifies the immune response to said determinant.

Affinity: The interaction of a ligands with its binding site can be characterized in terms of a binding affinity. In general, high affinity ligand binding results from greater intermolecular force between the ligand and its receptor while low affinity ligand binding involves less intermolecular force between the ligand and its receptor. In general, high affinity binding involves a longer residence time for the ligand at its receptor binding site than is the case for low affinity binding. High affinity binding of ligands to receptors is often physiologically important when some of the binding energy can be used to cause a conformational change in the receptor, resulting in altered behavior of an associated ion channel or enzyme. A ligand that can bind to a receptor, alter the function of the receptor and trigger a physiological response is called an agonist for that receptor. Agonist binding to a receptor can be characterized both in terms of how much physiological response can be triggered and the concentration of the agonist that is required to produce the physiological response. High affinity ligand binding implies that a relatively low concentration of a ligand is adequate to maximally occupy a ligand binding site and trigger a physiological response. Low affinity binding implies that a relatively high concentration of a ligand is required before the binding site is maximally occupied and the maximum physiological response to the ligand is achieved. Ligand binding is often characterized in terms of the concentration of ligand at which half of the receptor binding sites are occupied, known as the dissociation constant (kd). Affinity is also the strength of binding between receptors and their ligands, for example between an antibody and its antigen.

Agonist: An agonist is a compound capable of increasing or effecting the activity of a receptor.

Antagonist: An antagonist is in this case synonymous with an inhibitor. An antagonist is a compound capable of decreasing the activity of an effector such as a receptor.

Specifically, an agonist or antagonist against SorLA or the GDNF-family ligand receptors (GFRα1-4) may be binding to their extracellular domain, e.g. the domain that mediates the specific binding between SorLA and the GDNF-family ligand receptors (e.g. as given in SEQ ID NO 5 or 7). Agents directed able to modulate the interaction between the SorLA and the GDNF-family ligand receptors may include soluble fragments of the SorLA or GDNF-family ligand receptors, antibodies directed to each of the recptors, natural binding partners such as GDNF or Neurotensin, or synthetic small organic compounds.

Antibody: The term "antibody" as referred to herein includes whole antibodies and any antigen binding fragment (i.e., "antigen-binding portion") or single chain thereof.

"A whole antibody" refers to a glycoprotein comprising at least two heavy (H) chains and two light (L) chains inter-connected by disulfide bonds, or an antigen binding portion thereof. Each heavy chain is comprised of a heavy chain variable region (abbreviated herein as V_{H}) and a heavy chain constant region (abbreviated herein as C_{H}). Each light chain is comprised of a light chain variable region (abbreviated herein as VL) and a light chain constant region (abbreviated herein as C_{L}). The V_{H} and V_{L} regions can be further subdivided into regions of hypervariability, termed complementarity determining regions (CDRs), interspersed with regions that are more conserved, termed framework regions (FRs). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system.

The term "antigen-binding portion" of an antibody, as used herein, refers to one or more fragments of an antibody that retain the ability to specifically bind to an antigen. It has been shown that the antigen-binding function of an antibody can be performed by fragme ts of a full-length antibody. Examples of binding fragments encompassed within the term "antigen-binding portion" of an antibody include (i) a Fab fragment, a monovalent fragment consisting of the V_{L}, V_{H}, C_{L} and C_{H1} domains; (ii) a F(ab')2 fragment, a bivalent fragment comprising two Fab fragments linked by a disulfide bridge at the hinge region; (iii) a Fd fragment consisting of the V_{H} and C_{H1} domains; (iv) a Fv fragment consisting of the V_{L} and V_{H} domains of a single arm of an antibody, (v) a dAb fragment (Ward et al., (1989) Nature 341:544-546), which consists of a V_{H} domain; (vi) an isolated complementarity determining region (CDR),
and (vii) a combination of two or more isolated CDRs which may optionally be joined by a synthetic linker. Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); see e.g., Bird et al. (1988) Science 242:423-426; and Huston et al. (1988) Proc. Natl. Acad. Sci. USA 85:5879-5883).

Such single chain antibodies are also intended to be encompassed within the term "antigen-binding portion" of an antibody.

A further example of an antigen binding-domain is immunoglobulin fusion proteins comprising (i) a binding domain polypeptide that is fused to an immunoglobulin hinge region polypeptide, (ii) an immunoglobulin heavy chain CH2 constant region fused to the hinge region, and (iii) an immunoglobulin heavy chain CH3 constant region fused to the CH2 constant region. The binding domain polypeptide can be a heavy chain variable region or a light chain variable region. Such binding-domain immunoglobulin fusion proteins are further disclosed in US 2003/0118592 and US 2003/0133939 (both incorporated by reference in their entirety).

These antibody fragments are obtained using conventional techniques known to those with skill in the art, and the fragments are screened for utility in the same manner as are intact antibodies.

The term "epitope" means a protein determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

The term "human antibody", as used herein, is intended to include antibodies having variable and constant regions derived from human germline immunoglobulin sequences. The human antibodies of the invention may include amino acid residues not encoded by human germline immunoglobulin sequences (e.g., mutations introduced by random or site-specific mutagenesis in vitro or by somatic mutation in vivo). However, the term "human antibody", as used herein, is not intended to include antibodies in which CDR sequences derived from the germline of another mammalian species, such as a mouse, have been grafted onto human framework sequences.

The term "recombinant human antibody", as used herein, includes all human antibodies that are prepared, expressed, created or isolated by recombinant means, such as (a) antibodies isolated from an animal (e.g., a mouse) that is transgenic or transchromosomal for human immunoglobulin genes or a hybridoma prepared therefrom, (b) antibodies isolated from a host cell transformed to express the antibody, e.g., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable and constant regions derived from human germline immunoglobulin sequences. In certain embodiments, however, such recombinant human antibodies can be subjected to in vitro mutagenesis (or, when an animal transgenic for human Ig sequences is used, in vivo somatic mutagenesis) also called affinity maturation and thus the amino acid sequences of the V_{H} and V_{L} regions of the recombinant antibodies are sequences that, while derived from and related to human germline V_{H} and V_{L} sequences, may not naturally exist within the human antibody germline repertoire in vivo.

As used herein, "specific binding"' refers to antibody binding to a predetermined antigen/epitope. Typically, the antibody binds with an affinity corresponding to a K_{D} of about 10⁻⁷ M or less, such as about 10⁻⁸ M or less, such as about 10⁻⁹ M or less, about 10⁻¹⁰ M or less, or about 10⁻¹¹ M or even less, when measured as apparent affinities based on IC₅₀ values in FACS, and binds to the predetermined antigen with an affinity corresponding to a K_{D} that is at least ten-fold lower, such as at least 100-fold lower than its affinity for binding to a non-specific antigen (e.g., BSA, casein) other than the predetermined antigen or a closely-related antigen.

Antibody Classes: Depending on the amino acid sequences of the constant domain of their heavy chains, immunoglobulins can be assigned to different classes. There are at least five major classes of immunoglobulins: IgA, IgD, IgE, IgG and IgM, and several of these may be further divided into subclasses (isotypes), e.g. IgG-1, IgG-2, IgG-3 and IgG-4; IgA-1 and IgA-2. The heavy chains constant domains that correspond to the different classes of immunoglobulins are called alpha (a), delta (d), epsilon (e), gamma (g) and mu (µ), respectively. The light chains of antibodies can be assigned to one of two clearly distinct types, called kappa (k) and lambda (I), based on the amino sequences of their constant domain. The subunit structures and three-dimensional configurations of different classes of immunoglobulins are well known.

Chimeric antibody: An antibody in which the variable regions are from one species of animal and the constant regions are from another species of animal. For example, a chimeric antibody can be an antibody having variable regions which derive from a mouse monoclonal antibody and constant regions which are human.

Complementarity determining region or CDR: Regions in the V-domains of an antibody that together form the antibody recognizing and binding domain.

Constant Region or constant domain or C-domain: Constant regions are those structural portions of an antibody molecule comprising amino acid residue sequences within a given isotype which may contain conservative substitutions therein. Exemplary heavy chain immunoglobulin constant regions are those portions of an immunoglobulin molecule known in the art as CH1, CH2, CH3, CH4 and CH5. An exemplary light chain immunoglobulin constant region is that portion of an immunoglobulin molecule known in the art as CL.

Human antibody framework: A molecule having an antigen binding site and essentially all remaining immunoglobulin-derived parts of the molecule derived from a human immunoglobulin.

Humanised antibody framework: A molecule having an antigen binding site derived from an immunoglobulin from a non-human species, whereas some or all of the remaining immunoglobulin-derived parts of the molecule is derived from a human immunoglobulin. The antigen binding site may comprise: either a complete variable domain from the non-human immunoglobulin fused onto one or more human constant domains; or one or more of the complementarity determining regions (CDRs) grafted onto appropriate human framework regions in the variable domain. In a humanized antibody, the CDRs can be from a mouse monoclonal antibody and the other regions of the antibody are human.

Immunoglobulin: The serum antibodies, including IgG, IgM, IgA, IgE and IgD.

Immunoglobulin isotypes: The names given to the Ig which have different H chains, the names are IgG (IgG1,2,3,4), IgM, IgA (IgA1,2), slgA, IgE, IgD.

Immunologically distinct: The phrase immunologically distinct refers to the ability to distinguish between two polypeptides on the ability of an antibody to specifically bind one of the polypeptides and not specifically bind the other polypeptide.

Monoclonal Antibody: The phrase monoclonal antibody in its various grammatical forms refers to a population of antibody molecules that contains only one species of antibody combining site capable of immunoreacting with a particular antigen. A monoclonal antibody thus typically displays a single binding affinity for any antigen with which it immunoreacts. A monoclonal antibody may contain an antibody molecule having a plurality of antibody combining sites, each immunospecific for a different antigen, e.g., a bispecific monoclonal antibody.

Polyclonal antibody: Polyclonal antibodies are a mixture of antibody molecules recognizing a specific given antigen, hence polyclonal antibodies may recognize different epitopes within said antigen.

Binding: The term "binding" or "associated with" refers to a condition of proximity between chemical entities or compounds, or portions thereof. The association may be non-covalent-wherein the juxtaposition is energetically favoured by hydrogen bonding or van der Waals or electrostatic interactions-or it may be covalent.

Binding site: The term "binding site" or "binding pocket", as used herein, refers to a region of a molecule or molecular complex that, as a result of its shape, favourably associates with another molecule, molecular complex, chemical entity or compound. As used herein, the pocket comprises at least a deep cavity and, optionally a shallow cavity.

Fragments: The polypeptide fragments according to the present invention, including any functional equivalents thereof, may in one embodiment comprise less than 30 amino acid residues, for example less than 25 amino acid, less than 15 amino acids or less than 10 amino acids. Thus, it is contemplated that a fragment may e.g. comprise from about 2 to about 30 amino acids, or from about 2 to about 25, about 15 or about 10 amino acids, respectably.

GDNF-family ligand receptors is intended to include the receptors named GFRα1, 2, 3, and/or 4. The definition also includes isoforms thereof and, if specified, fragments or domains thereof as specified in e.g. SEQ ID NO 4 to 12.

SorLA is intended to include the SorLA receptor or fragments or domains thereof, as specified in e.g. SEQ ID NO 1 to 3.

Homology: The homology between amino acid sequences may be calculated using well known scoring matrices such as any one of BLOSUM 30, BLOSUM 40, BLOSUM 45, BLOSUM 50, BLOSUM 55, BLOSUM 60, BLOSUM 62, BLOSUM 65, BLOSUM 70, BLOSUM 75, BLOSUM 80, BLOSUM 85, and BLOSUM 90.

Ligand: a substance, compound or biomolecule such as a protein including receptors, that is able to bind to and form a complex with (a second) biomolecule to serve a biological purpose. In a narrower sense, it is a signal triggering molecule binding to a site on a target protein, by intermolecular forces such as ionic bonds, hydrogen bonds and Van der Waals forces. The docking (association) is usually reversible (dissociation). Actual irreversible covalent binding between a ligand and its target molecule is rare in biological systems. As opposed to the meaning in metalorganic and inorganic chemistry, it is irrelevant, whether or not the ligand actually binds at a metal site, as it is the case in hemoglobin. Ligand binding to receptors may alter the chemical conformation, i.e. the three dimensional shape of the receptor protein. The conformational state of a receptor protein determines the functional state of a receptor. The tendency or strength of binding is called affinity. Ligands include substrates, inhibitors, activators, non-self receptors, co-receptors and neurotransmitters. Radioligands are radioisotope labeled compounds and used in vivo as tracers in PET studies and for in vitro binding studies.

Pharmaceutical agent: The terms "pharmaceutical agent" or "drug" or "medicament"or "agent" refer to any therapeutic or prophylactic agent which may be used in the treatment (including the prevention, diagnosis, alleviation, or cure) of a malady, affliction, condition, disease or injury in a patient. Therapeutically useful genetic determinants, peptides, polypeptides and polynucleotides may be included within the meaning of the term pharmaceutical or drug. As defined herein, a "therapeutic agent", "pharmaceutical agent" or "drug" or "medicament" or "agent" is a type of bioactive agent.

Pharmaceutical composition: or composition refers to any chemical or biological material, compound, or composition capable of inducing a desired therapeutic effect when properly administered to a patient.

Polypeptide: The term "polypeptide" as used herein refers to a molecule comprising at least two amino acids. The amino acids may be natural or synthetic. "

The term "polypeptide" is also intended to include proteins, i.e. functional biomolecules comprising at least one polypeptide; when comprising at least two polypeptides, these may form complexes, be covalently linked or may be non-covalently.

Sequence identity: Sequence identity is determined in one embodiment by utilising fragments comprising at least 25 contiguous amino acids and having an amino acid sequence which is at least 80%, such as 85%, for example 90%, such as 95%, for example 99% identical to the amino acid sequence the protein in question, wherein the percent identity is determined with the algorithm GAP, BESTFIT, or FASTA in the Wisconsin Genetics Software Package Release 7.0, using default gap weights.

The invention provides evidence that SorLA - independently of GDNF - binds GFRα1 with a remarkably high affinity and that the two are efficiently coprecipitated from transfected cells (Fig. 6). When co-expressed in cells, SorLA conveys internalization and down-regulation of GFRα1, and mediates its retrograde transport to perinuclear (Golgi-)compartments thereby avoiding lysosomal degradation (Fig. 6). Notably, similar results with GFRα2, 3, and -4 suggest that SorLA may interact with all GFRα types, and that the functional implications described below may in fact concern not just GDNF but also neuturin, persephin and artemin. Moreover, expression of SorLA profoundly affects GDNF induction of Ret signalling, *i.e.* in Ret and GFRα1 positive cells responding to increasing concentrations of GDNF, overexpression of SorLA markedly inhibits Ret phosphorylation (Fig. 9). Studies of function performed on neuroblastoma cells and cultured neurons are in line with these results. Thus, the presence of SorLA hampers GDNF-induced survival, proliferation, and differentiation of neuroblastoma cells (Fig. 9), and the GDNF-induced survival of primary dopaminergic neurons is significantly enhanced when SorLA is functionally blocked by anti-SorLA antibodies (Fig. 12).

The inventors therefore propose that the interaction between SorLA and GDNF-family ligand receptors, such as GFRα1, are a key regulatory element in GDNF signalling, and that drugs (peptides, proteins, synthetic, small organic compounds) targeting the responsible binding sites in GDNF-family ligand receptors and/or SorLA can be used to promote or hamper GDNF functions by abrogating the GDNF-family ligand receptors binding to SorLA. The invention therefore relates to a method to hamper or reduce the survival, proliferation, and differentiation of neuroblastoma cells and, in another embodiment, increase the survival of neurons, such as e.g. dopaminergic neurons.

It is envisaged that various disease can be treated using these agents or drugs which are able to modulate the interaction between SorLA and GDNF-family ligand receptors and wherein the loss of neurons, such as e.g. dopaminergic neurons, is to be reversed or reduced and/or wherein the differentiation, proliferation and/or survival of neuroblastoma cells is to be reduced. These disease include attention deficit and hyperactivity disorder (ADHD), drug abuse and axiety disorder.

The inventors show that targeting of a GFRα:SorLA complex represents a completely new approach to the treatment of GFL-phenotypes in vivo - including behavioural phenotypes like ADHD. Such strategy has a number of advantages over the above mentioned ongoing clinical and preclinical trials. For example, instead of delivering the -36 kDa GDNF family ligand dimer directly, the inventors propose the generation of a small molecule agonist or antagonist that crosses the blood-brain barrier and specifically modulates the interaction between SorLA and a GFRα receptor, thereby increasing the biological activity of endogenous GDNF family ligands.

Different agents can be envisaged to have these modulator effects on the SorLA and GDNF-family ligand receptor interaction. In particular, the agent used to modulate the interaction between the SorLA and GDNF-family ligand receptors is selected antibodies.

The inventors further find that SorLA-/- transgenic mice exhibit a behavioural phenotype characterized by attention deficits and hyperactivity (Fig. 14), suggestive of altered dopaminergic activity - and an ADHD-like phenotype. Preliminary experiments using amphetamine-treatment of wt and transgenic mice support this hypothesis, inasmuch as SorLA -/- mice appear to be insensitive to amphetamine.

According to a particular embodiment the invention relates to a method to increase the extracellular levels of GDNF in the brain of a patient in the need thereof by modulating or inhibiting the interaction between SorLA and GFRα1, e.g. by inhibiting the internalisation and/or the degradation of GDNF is inhibited.

It is envisaged the this method can increases the survival of neurons, such as dopaminergic neurons by modulating the interaction between SorLA and GFRα1 and thus be suitable for treating diseases such as attention deficit and hyperactivity disorder (ADHD), drug abuse and axiety disorder.

An agent suitable for modulating or inhibiting the interaction between the SorLA and GFRα1 is an antibody directed against SorLA or GFRα1. This antibody preferably binds the extracellular domain of GFRα1 and/or SorLA comprising e.g. binding sites such as SEQ ID NO: 3, SEQ ID NO: 4, SEQ ID NO:5, SEQ ID NO: 6 or SEQ ID NO: 7.

The antibody may be polyclonal or monoclonal such as a humanized, chimeric, or single-chain antibody.

### Antibodies

Antibodies which bind to the same receptor targets as SorLA or the GDNF-family ligand receptors, such as GFRα1-4 of the invention can be prepared using an intact polypeptide or fragments containing small peptides of interest as the immunising antigen. The polypeptide used to immunise an animal may be obtained by recombinant DNA techniques or by chemical synthesis, and may optionally be conjugated to a carrier protein.

The preparation of polyclonal and monoclonal antibodies is well known in the art.

Polyclonal antibodies may in particular be obtained as described by, e.g., Green et al.,: "Production of Polyclonal Antisera" in Immunochemical 5 Protocols (Manson, Ed.); Humana Press, 1992, pages 1-5; by Coligan et al.,: "Production of Polyclonal Antisera in Rabbits, Rats, Mice and Hamsters" in Current Protocols in Immunology, 1992, Section 2.4.1, and by Ed Harlow and David Lane (Eds.) in "Antibodies; A laboratory manual" Cold Spring Harbor Lab. Press 1988. Monoclonal antibodiesmay in particular be obtained as described by, e.g., Kohler & Milstein, Nature, 1975, 256:495; Coligan et al., in Current Protocols in Immunology, 1992, Sections 2.5.1 -2.6.7; and Harlow et al., in Antibodies: A Laboratory Manual; Cold Spring Harbor, Pub., 1988, page 726 (all incorporated by reference in their entirety).

Briefly, monoclonal antibodies may be obtained by injecting, e.g., mice with a composition comprising an antigen, verifying the presence of antibody production by removing a serum sample, removing the spleen to obtain B lymphocytes, fusing the B lymphocytes with myeloma cells to produce hybridomas, cloning the hybridomas, selecting positive clones that produce the antibodies to the antigen, and isolating the antibodies from the hybridoma cultures.

Monoclonal antibodies can be isolated and purified from hybridoma cultures by a variety of well-established techniques, including affinity chromatography with protein A Sepharose, size-exclusion chromatography, and ion-exchange chromatography, see. e.g. Coligan et al. in Current Protocols in Immunology, 1992, Sections 2.7.1 -2.7.12, and Sections 2.9.1 - 2.9.3; and Barnes et al.: "Purification of Immunoglobulin 25 G (IgG)" in Methods in Molecular Biology; Humana Press, 1992, Vol. 10, Pages 79-104(all incorporated by reference in their entirety). Polyclonal or monoclonal antibodies may optionally be further purified, e.g. by binding to and elution from a matrix to which the polypeptide, to which the antibodies were raised, is bound.

Antibodies against GFRα1-4 are commercially available for e.g. R&D Systems, such as Human GDNF MAb (Clone 27106), Mouse IgG1 Human GFR alpha-4 MAb (Clone 215725), Mouse IgG1 Human GFR alpha-2 MAb (Clone 129030), Mouse IgG2B Human GFR alpha-3 MAb (Clone 111004), Mouse IgG1 Human GFR alpha-1 MAb (Clone 260714), Mouse IgG1 Human/Rat GDNF Affinity Purified Polyclonal Ab, Goat IgG Human GFR alpha-4 Affinity Purified Polyclonal Ab, Goat IgG Human GFR alpha-1 Affinity Purified Polyclonal Ab, Goat IgG Human GFR alpha-2 Affinity Purified Polyclonal Ab, Goat IgG Human GFR alpha-3 Affinity Purified Polyclonal Ab, Goat IgG

Furthermore, the inventors have generated rabbit polyclonal antibodies raised against the entire extracellular domain of human SorLA. The SorLA extracellular domain was expressed in CHO cells and purified from culture supernatant using affinity chromatography.

These antibodies can function as a SorLA antagonist as demonstrated in figures 9 and 12.

The inventors have in a similar manner generated a panel of mouse monoclonal antibodies raised against the entire extracellular domain of human SorLA. These can be selected based on their ability to function as a SorLA agonist or antagonist. The paratope of the selected antibody can then be cloned and a humanized antibody can be generated.

According to another embodiment the invention relates to an antibody having an epitope on the extracellular domain of SorLA, in particular the sequences comprising the N-terminal Vpsp10p-domain (SEQ ID NO 3), SEQ ID No 1 or 2 or at least 80% sequence identity to SEQ ID NO 1, 2 or 3, such as at least 85%, 90%, 95% or 98% sequence identity to SEQ ID NO 1, 2 or 3 is able to have similar or related effects on the interactions.

In one embodiment, the antibody as defined herein above, is selected from the group consisting of: polyclonal antibodies, monoclonal antibodies, humanised antibodies, single chain antibodies, recombinant antibodies., chimeric antibodies or just an antigen portion thereof.

### Pharmaceutical composition and administration forms

The main routes of drug delivery, in the treatment method are intravenous, oral, and topical. Other drug-administration methods, such as subcutaneous injection or via inhalation, which are effective to deliver the drug to a target site or to introduce the drug into the bloodstream, are also contemplated.

As the majority of compounds of the invention are proteins the most common way of administration is intravenous, intramuscular or subcutaneous administration, even though administration through intranasal application is also well described in the literature.

Appropriate dosage forms for such administration may be prepared by conventional techniques.

The compounds according to the invention may be administered as a single active agent or with at least one other active agent.

### Formulations

Whilst it is possible for the compounds of the present invention to be administered as the raw chemical, it is preferred to present them in the form of a pharmaceutical formulation.

Accordingly, the present invention further provides a pharmaceutical formulation, for medicinal application, which comprises the compound of the present invention and a pharmaceutically acceptable carrier or diluent.

In some embodiments of the invention is provided in a form of an antibody. A pharmaceutical formulation includes this antibody and an adjuvant.

Non-limiting examples of suitable adjuvants are selected from the group consisting of an immune targeting adjuvant; an immune modulating adjuvant such as a toxin, a cytokine, and a mycobacterial derivative; an oil formulation ; a polymer; a micelle forming adjuvant; a saponin ; an immunostimulating complex matrix (IS-COM matrix) ; a particle ; DDA ; aluminium adjuvants; DNA adjuvants ; y-inulin; and an encapsulating adjuvant.

The application of adjuvants include use of agents such as aluminum hydroxide or phosphate (alum).

According to the invention DDA (dimethyldioctadecylammonium bromide) is also a candidate for an adjuvant as is DNA and y-inulin, but also Freund's complete and incomplete adjuvants as well as quillaja saponins such as QuilA and QS21 are interesting as is RIBI. Further possibilities are monophos-phoryl lipid A (MPL), the above mentioned C3 and C3d, and mu- ramyl dipeptide (MDP).

Liposome formulations are also known to confer adjuvant effects, and therefore liposome adjuvants are preferred according to the invention.

Also immunostimulating complex matrix type (ISCOMO matrix) ad juvants are can be used according to the invention.

Details relating to composition and use of immunostimulating complexes can e. g. be found in Morein B et al., 1995, Clin. Immunother. 3: 461-475 as well as Barr IG and Mitchell GF, 1996, Immunol. and Cell Biol. 74: 8-25 (both incorporated by reference herein).

The compounds of the present invention may be formulated for parenteral administration (e.g., by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, for example solutions in aqueous polyethylene glycol. Examples of oily or nonaqueous carriers, diluents, solvents or vehicles include propylene glycol, polyethylene glycol, vegetable oils (e.g., olive oil), and injectable organic esters (e.g., ethyl oleate), and may contain formulatory agents such as preserving, wetting, emulsifying or suspending, stabilizing and/or dispersing agents.

Alternatively, the active ingredient may be in powder form, obtained by aseptic isolation of sterile solid or by lyophilisation from solution for constitution before use with a suitable vehicle, e.g., sterile, pyrogen-free water.

Oils useful in parenteral formulations include petroleum, animal, vegetable, or synthetic oils. Specific examples of oils useful in such formulations include peanut, 35 soybean, sesame, cottonseed, corn, olive, petrolatum, and mineral. Suitable fatty acids for use in parenteral formulations include oleic acid, stearic acid, and isostearic acid. Ethyl oleate and isopropyl myristate are examples of suitable fatty acid esters.

Suitable soaps for use in parenteral formulations include fatty alkali metal, ammonium, and triethanolamine salts, and suitable detergents include (a) cationic detergents such as, for example, dimethyl dialkyl ammonium halides, and alkyl pyridinium halides; (b) anionic detergents such as, for example, alkyl, aryl, and olefin sulfonates, alkyl, olefin, ether, and monoglyceride sulfates, and sulfosuccinates, (c) nonionic detergents such as, for example, fatty amine oxides, fatty acid alkanolamides, and polyoxyethylenepolypropylene copolymers, (d) amphoteric detergents such as, for example, alkyl-.beta.-aminopropionates, and 2-alkylimidazoline quaternary ammonium salts, and (e) mixtures thereof.

The parenteral formulations typically will contain from about 0.5 to about 25% by weight of the active ingredient in solution. Preservatives and buffers may be used. In order to minimize or eliminate irritation at the site of injection, such compositions may contain one or more nonionic surfactants having a hydrophile-lipophile balance (HLB) of from about 12 to about 17. The quantity of surfactant in such formulations will typically range from about 5to about 15% by weight. Suitable surfactants include polyethylene sorbitan fatty acid esters, such as sorbitan monooleate and the high molecular weight adducts of ethylene oxide with a hydrophobic base, formed by the condensation of propylene oxide with propylene glycol. The parenteral formulations can be presented in unit-dose or multi-dose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets of the kind previously described.

### Transdermal Delivery

The pharmaceutical agent-chemical modifier complexes described herein can be administered transdermally. Transdermal administration typically involves the delivery of a pharmaceutical agent for percutaneous passage of the drug into the systemic circulation of the patient. The skin sites include anatomic regions for transdermally administering the drug and include the forearm, abdomen, chest, back, buttock, mastoidal area, and the like.

Transdermal delivery is accomplished by exposing a source of the complex to a patient's skin for an extended period of time. Transdermal patches have the added advantage of providing controlled delivery of a pharmaceutical agent-chemical modifier complex to the body. See Transdermal Drug Delivery: Developmental Issues and Research Initiatives, Hadgraft and Guy (eds.), Marcel Dekker, Inc., (1989); Controlled Drug Delivery: Fundamentals and Applications, Robinson and Lee (eds.), Marcel Dekker Inc., (1987); and Transdermal Delivery of Drugs, Vols. 1- 3, Kydonieus and Berner (eds.), CRC Press, (1987) (all incoporated by reference in their entirety). Such dosage forms can be made by dissolving, dispersing, or otherwise incorporating the pharmaceutical agent-chemical modifier complex in a proper medium, such as an elastomeric matrix material. Absorption enhancers can also be used to increase the flux of the compound across the skin. The rate of such flux can be controlled by either providing a rate-controlling membrane or dispersing the compound in a polymer matrix or gel.

### Passive Transdermal Drug Delivery

A variety of types of transdermal patches will find use in the methods described herein. For example, a simple adhesive patch can be prepared from a backing material and an acrylate adhesive. The pharmaceutical agent-chemical modifier complex and any enhancer are formulated into the adhesive casting solution and allowed to mix thoroughly. The solution is cast directly onto the backing material and the casting solvent is evaporated in an oven, leaving an dhesive film. The release liner can be attached to complete the system.

Alternatively, a polyurethane matrix patch can be employed to deliver the pharmaceutical agent-chemical modifier complex. The layers of this patch comprise a backing, a polyurethane drug/enhancer matrix, a membrane, an adhesive, and a release liner. The polyurethane matrix is prepared using a room temperature curing polyurethane prepolymer. Addition of water, alcohol, and complex to the prepolymer results in the formation of a tacky firm elastomer that can be directly cast only the backing material.

A further embodiment of this invention will utilize a hydrogel matrix patch. Typically, the hydrogel matrix will comprise alcohol, water, drug, and several hydrophilic polymers. This hydrogel matrix can be incorporated into a transdermal patch between the backing and the adhesive layer.

The liquid reservoir patch will also find use in the methods described herein. This patch comprises an impermeable or semipermeable, heat sealable backing material, a heat sealable membrane, an acrylate based pressure sensitive skin adhesive, and a siliconized release liner. The backing is heat sealed to the membrane to form a reservoir which can then be filled with a solution of the complex, enhancers, gelling agent, and other excipients.

Foam matrix patches are similar in design and components to the liquid reservoir system, except that the gelled pharmaceutical agent-chemical modifier solution is constrained in a thin foam layer, typically a polyurethane. This foam layer is situated between the backing and the membrane which have been heat sealed at the periphery of the patch.

For passive delivery systems, the rate of release is typically controlled by a membrane placed between the reservoir and the skin, by diffusion from a monolithic device, or by the skin itself serving as a rate-controlling barrier in the delivery system. See U.S. Pat. Nos. 4,816,258; 4,927,408; 4,904,475; 4,588,580, 4,788,062;and the like (all incorporated by reference in their entirety). The rate of drug delivery will be dependent, in part, upon the nature of the membrane. For example, the rate of drug delivery across membranes within the body is generally higher than across dermal barriers. The rate at which the complex is delivered from the device to the membrane is most advantageously controlled by the use of rate-limiting membranes which are placed between the reservoir and the skin. Assuming that the skin is sufficiently permeable to the complex (i.e., absorption through the skin is greater than the rate of passage through the membrane), the membrane will serve to control the dosage rate experienced by the patient.

Suitable permeable membrane materials may be selected based on the desired degree of permeability, the nature of the complex, and the mechanicalconsiderations related to constructing the device. Exemplary permeable membrane materials include a wide variety of natural and synthetic polymers, such as polydimethylsiloxanes (silicone rubbers), ethylenevinylacetate copolymer (EVA), polyurethanes, polyurethane-polyether copolymers, polyethylenes, polyamides, polyvinylchlorides (PVC), polypropylenes, polycarbonates, polytetrafluoroethylenes (PTFE), cellulosic materials, e.g., cellulose triacetate and cellulose nitrate/acetate, and hydrogels, e.g., 2-hydroxyethylmethacrylate (HEMA).

Other items may be contained in the device, such as other conventional components of therapeutic products, depending upon the desired device characteristics. For example, the compositions according to this invention may also include one or more preservatives or bacteriostatic agents, e.g., methyl hydroxybenzoate, propyl hydroxybenzoate, chlorocresol, benzalkonium chlorides, and the like. These pharmaceutical compositions also can contain other active ingredients such as antimicrobial agents, particularly antibiotics, anesthetics, analgesics, and antipruritic agents.

The compounds of the present invention may be formulated for administration as suppositories. A low melting wax, such as a mixture of fatty acid glycerides or cocoa butter is first melted and the active component is dispersed homogeneously, for example, by stirring. The molten homogeneous mixture is then poured into convenient sized molds, allowed to cool, and to solidify.

The active compound may be formulated into a suppository comprising, for example, about 0.5% to about 50% of a compound of the invention, disposed in a polyethylene glycol (PEG) carrier (e.g., PEG 1000 [96%] and PEG 4000 [4%]. The compounds of the present invention may be formulated for vaginal administration. Pessaries, tampons, creams, gels, pastes, foams or sprays containing in addition to the active ingredient such carriers as are known in the art to be appropriate.

The compounds of the present invention may be formulated for nasal administration.

The solutions or suspensions are applied directly to the nasal cavity by conventional means, for example with a dropper, pipette or spray. The formulations may be provided in a single or multidose form. In the latter case of a dropper or pipette this may be achieved by the patient administering an appropriate, predetermined volume of the solution or suspension. In the case of a spray this may be achieved for example by means of a metering atomizing spray pump.

The compounds of the present invention may be formulated for aerosol administration, particularly to the respiratory tract and including intranasal administration. The compound will generally have a small particle size for example of the order of 5 microns or less. Such a particle size may be obtained by means known in the art, for example by micronization. The active ingredient is provided in a pressurized pack with a suitable propellant such as a chlorofluorocarbon (CFC) for example dichlorodifluoromethane, trichlorofluoromethane, or dichlorotetrafluoroethane, carbon dioxide or other suitable gas. The aerosol may conveniently also contain a surfactant such as lecithin. The dose of drug may be controlled by a metered valve. Alternatively the active ingredients may be provided in a form of a dry powder, for example a powder mix of the compound in a suitable powder base such as lactose, starch, starch derivatives such as hydroxypropylmethyl cellulose and polyvinylpyrrolidine (PVP). The powder carrier will form a gel in the nasal cavity. The powder composition may be presented in unit dose form for example in capsules or cartridges of e.g., gelatin or blister packs from which the powder may be administered by means of an inhaler. When desired, formulations can be prepared with enteric coatings adapted for sustained or controlled release administration of the active ingredient. The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage 5 form can be a capsule, tablet, cachet, or lozenge itself, or it can be the appropriate number of any of these in packaged form.

In a the injection is intravenous, intramuscular, intraspinal, intraperitoneal, subcutaneous, a bolus or a continuous administration.

In one embodiment the pharmaceutical composition according to the present invention is administered at intervals of 30 minutes to 24 hours.

In a further embodiment the pharmaceutical composition according to the present invention is administered at intervals of 1 to 6 hours.

In a further embodiment the pharmaceutical composition according to the present invention is administered at intervals of 6 to 72 hours.

In another embodiment the pharmaceutical composition is administered at a dosage of between 10 µg to 500 mg per kg body mass.

The polypeptides and antibodies of the present invention may be administered in any manner, which is medically acceptable. This may include injections, by parenteral 10 routes such as intravenous, intravascular, intraarterial, subcutaneous, intramuscular, intratumor, intraperitoneal, intraventricular, intraepidural, intertracheal, intrathecal, intracerebroventricular, intercerebral, interpulmonary, or others as well as nasal, ophthalmic, rectal, or topical. Sustained release administration is also specifically included in the invention, by such means as depot injections or erodible implants.

Peroral administration is also conceivable provided the protein is protected against degradation in the stomach.

Administration may be by periodic injections of a bolus of the preparation, or may be made more continuous by intravenous or intraperitoneal administration from a reservoir which is external (e.g., an IV bag) or internal (e.g., a bioerodable implant, a bioartificial organ, a biocompatible capsule. See, e.g., U.S. Patents 4,407,957, 5,798,113, and 5,800,828, each incorporated herein by reference. Intrapulmonary delivery methods and apparatus are described, for example, in U.S. Patents 5,654,007, 5,780,014, and 5,814,607, each incorporated herein by reference. Apart from systemic delivery, delivery directly to the CNS behind the blood-brain or blood-retina barriers is also contemplated. Localised delivery may be by such means as delivery via a catheter to one or more arteries, such as the cerebral artery to the CNS. Methods for local pump-based delivery of protein formulations to the CNS are described in US 6,042,579 (Medtronic) (incoporated by reference in its entirety).

The term "pharmaceutically acceptable carrier" means one or more organic or inorganic ingredients, natural or synthetic, with which the polypeptides or antibodies are combined to facilitate its application. A suitable carrier includes sterile saline although other aqueous and non-aqueous isotonic sterile solutions and sterile suspensions known to be pharmaceutically acceptable are known to those of ordinary skill in the art.

An "effective amount" refers to that amount which is capable of ameliorating or delaying progression of the diseased, degenerative or damaged condition. An effective amount can be determined on an individual basis and will be based, in part, on consideration of the symptoms to be treated and results sought. An effective amount can be determined by one of ordinary skill in the art employing such factors and using no more than routine experimentation.

A liposome system may be any variety of unilamellar vesicles, multilamellar vesicles, or stable plurilamellar vesicles, and may be prepared and administered according to methods well known to those of skill in the art, for example in accordance with the teachings of United States Patents 5,169,637,4,762,915, 5,000,958 or 5,185,154 (all incoporated by reference in their entirety). In addition, it may be desirable to express the novel polypeptides of this invention, as well as other selected polypeptides, as lipoproteins, in order to enhance their binding to liposomes.

Various dosing regimes for systemic administration are contemplated. In one embodiment, methods of administering to a subject a formulation comprising the antibody or the polypeptides include administering said at a dosage of between 1 µg/kg to 30,000 µg/kg body weight of the subject, per dose.

In another embodiment, the dosage is between 10 µg/kg to 30,000 µg/kg body weight of th subject, per dose. In a further embodiment, the dosage is between 10 30 µg/kg to 10,000 µg/kg body weight of the subject, per dose. In a different embodiment, the dosage is between 5 µg/kg to 10,000 µg/kg body weight of the subject, per dose. In yet another embodiment, the dosage is between 25 µg/kg to 3,000 µg/kg body weight of the subject, per dose. In a most preferable embodiment, the dosage is between 50 µg/kg to 3,000 µg/kg body weight of the subject, per dose. Guidance as to particular dosages and methods of delivery is provided in the literature; see, for example, U.S. Pat. Nos. 4,657,760; 5,206,344; or 5,225,212 (all incoporated by reference in their entirety). It is anticipated that different formulations will be effective for different treatment compounds and different disorders, that administration targeting one organ or tissue, for example, may necessitate delivery in a manner different 5 from that to another organ or tissue.

### References

Andersen, O. M., J. Reiche, et al. (2005). "Neuronal sorting protein-related receptor sorLA/LR11 regulates processing of the amyloid precursor protein." Proc Natl Acad Sci U S A 102(38): 13461-6.
Beck, K. D., J. Valverde, et al. (1995). "Mesencephalic dopaminergic neurons protected by GDNF from axotomy-induced degeneration in the adult brain." Nature 373(6512): 339-41.
Cacalano G, Farinas I, Wang LC, Hagler K, Forgie A, Moore M, Armanini M, Phillips H, Ryan AM, Reichardt LF, Hynes M, Davies A, Rosenthal A (1998) GFRal-pha1 is an essential receptor component for GDNF in the developing nervous system and kidney. Neuron 21 (1): 53-62
Chauhan NB, Siegel GJ, Lee JM (2001) Depletion of glial cell line-derived neurotrophic factor in substantia nigra neurons of Parkinson's disease brain. J Chem Neuroanat 21(4): 277-288
Ciechanowski, P. S., W. J. Katon, et al. (2000). "Depression and diabetes: impact of depressive symptoms on adherence, function, and costs." Arch Intern Med 160(21): 3278-85.
Davie, C. A. (2008). "A review of Parkinson's disease." Br Med Bull 86: 109-27.
Dodson, S. E., M. Gearing, et al. (2006). "LR11/SorLA expression is reduced in sporadic Alzheimer disease but not in familial Alzheimer disease." J Neuropathol Exp Neurol 65(9): 866-72.
Durbec PL, Larsson-Blomberg LB, Schuchardt A, Costantini F, Pachnis V (1996) Common origin and developmental dependence on c-ret of subsets of enteric and sympathetic neuroblasts. Development 122(1): 349-358
Enomoto H, Araki T, Jackman A, Heuckeroth RO, Snider WD, Johnson EM, Jr., Milbrandt J (1998) GFR alpha1-deficient mice have deficits in the enteric nervous system and kidneys. Neuron 21(2): 317-324
Gash, D. M., G. A. Gerhardt, et al. (1998). "Effects of glial cell line-derived neurotrophic factor on the nigrostriatal dopamine system in rodents and nonhuman primates." Adv Pharmacol 42: 911-5.
Gill, S. S., N. K. Patel, et al. (2003). "Direct brain infusion of glial cell line-derived neurotrophic factor in Parkinson disease." Nat Med 9(5): 589-95.
Golden JP, Baloh RH, Kotzbauer PT, Lampe PA, Osborne PA, Milbrandt J, Johnson EM, Jr. (1998) Expression of neurturin, GDNF, and their receptors in the adult mouse CNS. J Comp Neurol 398(1): 139-150
Granholm AC, Reyland M, Albeck D, Sanders L, Gerhardt G, Hoernig G, Shen L, Westphal H, Hoffer B (2000) Glial cell line-derived neurotrophic factor is essential for postnatal survival of midbrain dopamine neurons. J Neurosci 20(9): 3182-3190
Jacobsen L, Madsen P, Nielsen MS, Geraerts WP, Gliemann J, Smit AB, Petersen CM (2002) The sorLA cytoplasmic domain interacts with GGA1 and -2 and defines minimum requirements for GGA binding. FEBS Lett 511(1-3): 155-158
Kanaki, T., H. Bujo, et al. (1998). "Developmental regulation of LR11 expression in murine brain." DNA Cell Biol 17(8): 647-57.
Kearns, C. M. and D. M. Gash (1995). "GDNF protects nigral dopamine neurons against 6-hydroxydopamine in vivo." Brain Res 672(1-2): 104-11.
Kramer ER, Aron L, Ramakers GM, Seitz S, Zhuang X, Beyer K, Smidt MP, Klein R (2007) Absence of Ret signaling in mice causes progressive and late degeneration of the nigrostriatal system. PLoS Biol 5(3): e39
Kupfer, D. J. (2005). "The increasing medical burden in bipolar disorder." Jama 293(20): 2528-30.
Lang, A. E., S. Gill, et al. (2006). "Randomized controlled trial of intraputamenal glial cell line-derived neurotrophic factor infusion in Parkinson disease." Ann Neurol 59(3): 459-66.
Lin, L.F. (1993) et al. GDNF: a glial cell-derived neurotrophic factor for midbrain dopaminergic neurons. Science May 21: 1130-2
Manji, H. K., W. C. Drevets, et al. (2001). "The cellular neurobiology of depression." Nat Med 7(5): 541-7.
Marcusson, E. G., B. F. Horazdovsky, et al. (1994). "The sorting receptor for yeast vacuolar carboxypeptidase Y is encoded by the VPS10 gene." Cell 77(4): 579-86.
Mazella, J., N. Zsurger, et al. (1998). "The 100-kDa neurotensin receptor is gp95/sortilin, a non-G-protein-coupled receptor." J Biol Chem 273(41): 26273-6
Michelson, D., C. Stratakis, et al. (1996). "Bone mineral density in women with depression." N Engl J Med 335(16): 1176-81.
Moore MW, Klein RD, Farinas I, Sauer H, Armanini M, Phillips H, Reichardt LF, Ryan AM, Carver-Moore K, Rosenthal A (1996) Renal and neuronal abnormalities in mice lacking GDNF. Nature 382(6586): 76-79
Motoi, Y., T. Aizawa, et al. (1999). "Neuronal localization of a novel mosaic apolipoprotein E receptor, LR11, in rat and human brain." Brain Res 833(2): 209-15.
Munck Petersen, C., M. S. Nielsen, et al. (1999). "Propeptide cleavage conditions sortilin/neurotensin receptor-3 for ligand binding." Embo J 18(3): 595-604.
Musselman, D. L., D. L. Evans, et al. (1998). "The relationship of depression to cardiovascular disease: epidemiology, biology, and treatment." Arch Gen Psychiatry 55(7): 580-92.
Nishino J, Mochida K, Ohfuji Y, Shimazaki T, Meno C, Ohishi S, Matsuda Y, Fujii H, Saijoh Y, Hamada H (1999) GFR alpha3, a component of the artemin receptor, is required for migration and survival of the superior cervical ganglion. Neuron 23(4): 725-736
Nutt, J. G., K. J. Burchiel, et al. (2003). "Randomized, double-blind trial of glial cell line-derived neurotrophic factor (GDNF) in PD." Neurology 60(1): 69-73.
Nykjaer, A., R. Lee, et al. (2004). "Sortilin is essential for proNGF-induced neuronal cell death." Nature 427(6977): 843-8.
Offe, K., S. E. Dodson, et al. (2006). "The lipoprotein receptor LR11 regulates amyloid beta production and amyloid precursor protein traffic in endosomal compartments." J Neurosci 26(5): 1596-603.
Rogaeva, E., Y. Meng, et al. (2007). "The neuronal sortilin-related receptor SORL1 is genetically associated with Alzheimer disease." Nat Genet 39(2): 168-77.
Rossi J, Luukko K, Poteryaev D, Laurikainen A, Sun YF, Laakso T, Eerikainen S, Tuominen R, Lakso M, Rauvala H, Arumae U, Pasternack M, Saarma M, Airaksinen MS (1999) Retarded growth and deficits in the enteric and parasympathetic nervous system in mice lacking GFR alpha2, a functional neurturin receptor. Neuron 22(2): 243-252
Sager, K. L., J. Wuu, et al. (2007). "Neuronal LR11/sorLA expression is reduced in mild cognitive impairment." Ann Neurol 62(6): 640-7
Sauer, H., C. Rosenblad, et al. (1995). "Glial cell line-derived neurotrophic factor but not transforming growth factor beta 3 prevents delayed degeneration of nigral dopaminergic neurons following striatal 6-hydroxydopamine lesion." Proc Natl Acad Sci U S A 92(19): 8935-9.
Scherzer, C. R., K. Offe, et al. (2004). "Loss of apolipoprotein E receptor LR11 in Alzheimer disease." Arch Neurol 61 (8): 1200-5.
Schulz, R., S. R. Beach, et al. (2000). "Association between depression and mortality in older adults: the Cardiovascular Health Study." Arch Intern Med 160(12): 1761-8.
Sian J, Gerlach M, Youdim MB, Riederer P (1999) Parkinson's disease: a major hypokinetic basal ganglia disorder. J Neural Transm 106(5-6): 443-476
Souza et al. (2010). J Psychiatr Res.
Spoelgen, R., C. A. von Arnim, et al. (2006). "Interaction of the cytosolic domains of sorLA/LR11 with the amyloid precursor protein (APP) and beta-secretase beta-site APP-cleaving enzyme." J Neurosci 26(2): 418-28.
Syed Z, Dudbridge F, Kent L (2007) An investigation of the neurotrophic factor genes GDNF, NGF, and NT3 in susceptibility to ADHD. Am J Med Genet B Neuropsychiatr Genet 144B(3): 375-378
Teng, H. K., K. K. Teng, et al. (2005). "ProBDNF induces neuronal apoptosis via activation of a receptor complex of p75NTR and sortilin." J Neurosci 25(22): 5455-63
Tomac AC, Grinberg A, Huang SP, Nosrat C, Wang Y, Borlongan C, Lin SZ, Chiang YH, Olson L, Westphal H, Hoffer BJ (2000) Glial cell line-derived neurotrophic factor receptor alpha1 availability regulates glial cell line-derived neurotrophic factor signaling: evidence from mice carrying one or two mutated alleles. Neuroscience 95(4): 1011-1023
Tomac, A., E. Lindqvist, et al. (1995). "Protection and repair of the nigrostriatal dopaminergic system by GDNF in vivo." Nature 373(6512): 335-9.
Westergaard, U. B., E. S. Sorensen, et al. (2004). "Functional organization of the sortilin Vps10p domain." J Biol Chem 279(48): 50221-9.
Williams et al. (2007). Schizophr Res
Zaman V, Boger HA, Granholm AC, Rohrer B, Moore A, Buhusi M, Gerhardt GA, Hoffer BJ, Middaugh LD (2008) The nigrostriatal dopamine system of aging GFRalpha-1 heterozygous mice: neurochemistry, morphology and behavior. Eur J Neurosci 28(8): 1557-1568
Zurn AD, Widmer HR, Aebischer P (2001) Sustained delivery of GDNF: towards a treatment for Parkinson's disease. Brain Res Brain Res Rev 36(2-3): 222-229.

### EXAMPLES

### Example 1: Demonstration of a SorLA:GFRα1 complex

HEK293 cells stably transfected with plasmids encoding SorLA and GFRα1 were crosslinked with DSP (Pierce) and subsequently lysed. The cell lysate was incubated with antibody against SorLA or GFRα1 (R&D Systems) bound to Gammabind beads (GE Healthcare). Precipitated complexes were eluted from the washed beads with SDS loading buffer. Western blot analysis revealed the presence of a SorLA: GFRα1 complex (Fig. 5A). The direct interaction of the extracellular domains of SorLA and GFRα1 was also demonstrated using surface plasmon resonance (Biacore, Sweden) using CaHBS as standard running buffer (10 mM HEPES, pH 7.4, 140 mM NaCl, 2mM CaCl₂, 1 mM EGTA, 0.005% Tween-20). A biosensor chip from Biacore (CM5, cat.no. BR-1000-14) was activated using the NHS/EDC method as described by the supplier followed by coating with SorLA (Fig. 5C).

### Example 2: Demonstration of a SorLA:GFRα2 complex

HEK293 cells stably transfected with plasmids encoding SorLA and GFRα2 were crosslinked with DSP (Pierce) and subsequently lysed. The cell lysate was incubated with antibody against GFRα2 (R&D Systems) bound to Gammabind beads (GE Healthcare). Precipitated complexes were eluted from the washed beads with SDS loading buffer. Western blot analysis revealed the presence of a SorLA: GFRα2 complex (Fig. 11A). The direct interaction of the extracellular domains of SorLA and GFRα2 was also demonstrated using surface plasmon resonance (Biacore, Sweden) using CaHBS as standard running buffer (10 mM HEPES, pH 7.4, 140 mM NaCl, 2mM CaCl₂, 1 mM EGTA, 0.005% Tween-20). A biosensor chip from Biacore (CM5, cat.no. BR-1000-14) was activated using the NHS/EDC method as described by the supplier followed by coating with SorLA.

### Example 3: Demonstration of a SorLA:GFRα3 complex

HEK293 cells stably transfected with plasmids encoding SorLA and GFRα3 were crosslinked with DSP (Pierce) and subsequently lysed. The cell lysate was incubated with antibody against GFRα3 (R&D Systems) bound to Gammabind beads (GE Healthcare). Precipitated complexes were eluted from the washed beads with SDS loading buffer. Western blot analysis revealed the presence of a SorLA: GFRα3 complex (Fig. 11A). The direct interaction of the extracellular domains of SorLA and GFRα3 was also demonstrated using surface plasmon resonance (Biacore, Sweden) using CaHBS as standard running buffer (10 mM HEPES, pH 7.4, 140 mM NaCl, 2mM CaCl₂, 1 mM EGTA, 0.005% Tween-20). A biosensor chip from Biacore (CM5, cat.no. BR-1000-14) was activated using the NHS/EDC method as described by the supplier followed by coating with SorLA.

### Example 4: Demonstration of a SorLA:GFRα4 complex

HEK293 cells stably transfected with plasmids encoding SorLA and GFRα4 were crosslinked with DSP (Pierce) and subsequently lysed. The cell lysate was incubated with antibody against GFRα4 (R&D Systems) bound to Gammabind beads (GE Healthcare). Precipitated complexes were eluted from the washed beads with SDS loading buffer. Western blot analysis revealed the presence of a SorLA: GFRα4 complex (Fig. 11A). The direct interaction of the extracellular domains of SorLA and GFRα4 was also demonstrated using surface plasmon resonance (Biacore, Sweden) using CaHBS as standard running buffer (10 mM HEPES, pH 7.4, 140 mM NaCl, 2mM CaCl₂, 1 mM EGTA, 0.005% Tween-20). A biosensor chip from Biacore (CM5, cat.no. BR-1000-14) was activated using the NHS/EDC method as described by the supplier followed by coating with SorLA.

### Example 5: Demonstration of a SorLA:GDNF complex

The direct interaction of the extracellular domains of SorLA and GDNF was demonstrated using surface plasmon resonance (Biacore, Sweden) using CaHBS as standard running buffer (10 mM HEPES, pH 7.4, 140 mM NaCl, 2mM CaCl₂, 1 mM EGTA, 0.005% Tween-20). A biosensor chip from Biacore (CM5, cat.no. BR-1000-14) was activated using the NHS/EDC method as described by the supplier followed by coating with SorLA (Fig. 3).

### Example 6: A cell based screening method for identifying receptor antagonists/agonists that modulates the by complexes comprising SorLA and GFRα1.

Determination of binding, internalization or signaling by members of the Vps10p domain receptor family can be performed in cellular systems. Cells expressing SorLA and GFRα1 and GDNF following e.g. transfection with plasmids encoding all three receptors, respectively, are incubated with a candidate agent (inhibitor/antagonist) compound. Said agent can e.g. represent an antibody against either one of the receptors, SorLA and GFRα1, binding ligands such as the SorLA propeptide or, fragments of the respective receptors. After incubation, the cells are washed, protein complexes crosslinked with dithiobis[succinimidylpropionate] (DSP, Pierce) and subsequently lysed in TNE buffer (10 mM Tris-HCl, 150 mM NaCl, 1 mM EDTA, 1% nonides P-40, pH. 8) containing proteinase inhibitors (Complete, Roche Applied Science, Switzerland). The cell lysate is incubated with antibody against SorLA or GFRα1 (R&D Systems) bound to sepharose beads (GE Healthcare). Precipitated complexes are eluted from the washed beads (acidic buffer and subsequent neutralization). Western blot analysis of SorLA and GFRα1 in the eluate reveals whether candidate compounds are able to inhibit SorLA:GFRα1 complex formation.

### Example 7: An in vitro assay for identifying agents disrupting the interaction of GFRα1 and/or GDNF with SorLA

Determination of direct binding of a ligand such as a small organic molecule, a peptide or a soluble receptor including but not limited to SorLA, GFRα1 and GDNF, to immobilized protein can be performed by e.g. surface plasmon resonance analysis (Biacore, Sweden) using CaHBS as standard running buffer (10 mM HEPES, pH 7.4, 140 mM NaCl, 2 mM CaCl₂, 1 mM EGTA, and 0.005% Tween-20). Such an agent could be derived from the N-terminal of mature GDNF (Fig. 17) or the GFRα1 domain 1 (Fig. 16). A biosensor chip from Biacore (CM5, cat.no. BR-1000-14) is activated using the NHS/EDC method as described by supplier followed by coating with SorLA. Several different approaches can be applied: Candidate agents can be identified by comparing the binding signal (response units) to a chip immobilized with one of the receptors and comparing this signal to an empty flow cell. In another approach, inhibition of an established ligand can be monitered in the absence or presence of putative inhibitors. The difference in the signal depicts the inhibitory potential of the antagonist. The data collected are analysed by fitting of sensorgrams for affinity estimations and inhibitory potential using the Biaevaluation version 3.1 program. The surface Plasmon resonance assay can easily be transform into other assays in which the Vps10p-domain receptor, the ligand or the putative inhibitor is immobilized on a solid phase. For instance, receptors can be immobilized in e.g. Maxisorp microtiter wells from Nunc (cat.no. 439454) by incubation for 16 h at 4°C in 50 mM Na-HCO₃, pH 9.6. After blocking using 5% bovine serum albumin (Sigma, cat.no. A9647) for 2 h at room temperature, the wells are washed three times with MB buffer (10 mM HEPES, pH 7.4, 140 mM NaCl, 2 20 mM CaCl₂, and 1 mM MgCl₂) before incubation with a labelled ligand (e.g. iodinated) in the absence or presence of a various concentrations of a candidate inhibitor. Following incubation (e.g. overnight at 4°C) and washing with MB buffer, bound radioactivity is released by adding 10% SDS. Nonspecific binding of tracer to wells coated only with bovine serum albumin is determined and subtracted from the values determined in the binding experiments. The binding data point can be fitted to binding equations using the Prism software from GraphPad, version 4. Likewise, the antagonist can be labelled and binding to the immobilized receptor directly measured. In yet another setup, the receptor, ligand or antagonist can be immobilized on scintillation beads and binding measured in a scintillation proximity assay in which the receptor-binding molecule has been labelled using radioactivity.

### Example 8: A cell based screening method for identifying agents capable of inhibiting SorLA

An antagonist directed against an entity of the SorLA:GFRa1:GDNF receptor complex may act as an inhibitor of the entire complex. Accordingly it is relevant to screen for agents capable of binding to e.g. the Vps10p-domain receptor entity. Such a metod is described in the present example. Determination of binding, internalization or signalling by members of the Vps10pdomain receptor family can be performed in cellular systems. Cells expressing one of the receptors, either endogenously or following e.g. transfection with a plasmid containing the cDNA of the receptor, are incubated with a radio-labeled ligand, in the absence and the presence respectively, of a candidate inhibitor/antagonist compound. After incubation, the cells are washed to remove unspecific binding and subsequently harvested. The degree of binding of the candidate antagonist/linhibitor to the receptor is determined by using a conventional radioligand assay well known to those skilled in the art. See e.g. Bylund and Toews (1993) Am J Physiol. 265(5 Pt 1):L421-9 entitled "Radioligand binding methods: practical guide and tips". Likewise, endocytosis/internalization may be determined as described in Nykjaer et al (1992) FEBS 300:13- and Nielsen et al (2001) EMBO J.

### Example 9: A cell based screening method for identifying agents capable of modulating retrograde sorting of a GFRa receptor by SorLA.

Surface localized GFRa receptor was labeled either by a fluorescent tag or fluorescent antibodies in cells expressing both SorLA and GFRa receptor, and incubated with a SorLA agonist/antagonist for 30 min. The amount of internalized GFRa receptor was subsequently evaluated using confocal microscopy as shown in Fig. 6 and Fig. 11.

### Example 10: A cell based screening method for identifying agents capable of increasing Ret phosphorylation

SY5Y neuroblastoma cells were stimulated by increasing concentrations of GDNF for defined periods of time in the absence or presence a SorLA antagonist or agonist.

Cells were lysed and the cell lysates were incubated with antibody against Ret (R&D Systems) coupled to Gammabind beads (GE Healthcare). Precipitated protein was eluted from the washed beads (acidic buffer and subsequent neutralization) and phosphorylated Ret was visualized by Western blotting using anti-phosphotyrosine (Milipore). (As shown in Fig. 9).

### Example 11: A cell based screening method for identifying agents capable of increasing cell survival

SY5Y neuroblastoma cells were harvested by trypsin-EDTA treatment and thereafter resuspended in DMEM without phenol red (LONZA) containing 1% glutamax and 1% P/S (penicillin and streptomycin). The cells were plated: 20.000 cells pr. well in a 96 well plate in a final volume of 50 µl. Next day, cells were stimulated by increasing concentrations of GDNF in the absence or presence of a SorLA antagonist or agonist. The final volume after addition of GDNF etc. was 100 µl. Each kind of sample was made as quadruplets. The cells were incubated for 72 hours in their normal incubator. After the 72 hours the MultiTox-Fluor Multiplex Cytotoxicity Assay reagents (Promega) were prepared and added to the cells as directed by supporting technical literature from manufacturer. Briefly, for each sample, 100 µl of assay buffer, 0.1 µl of GF-AFC substrate, and 0.1 µl of bis-AAF-R110 substrate were mixed and added to the well. After 1 min mixing at 240 rpm on an orbital shaker (Ika^{®}KS 260 Basic) the plate was incubated at 37° C for 30 min. The fluorescence was thereafter measured with a Wallac VICTOR3TM 1420 Multilabel Counter (Perkin ElmerTM Lifesciences). Viability was measured at 400nmEx/505nmEm. The signal is directly proportional to the survival.

Another way to test the survival is to grow the cells on coverslips instead of 96-well plate. After the 72 hours the cells could be fixed in 4% paraformaldehyde (PFA) in PBS for 30 min at room temperature and mounted onto slides using mounting medium containing DAPI. The DAPI stained cells could be counted directly in a fluorescence microscope.
(As shown in Fig. 9D).

### Example 12: Modulation of GDNF-family ligand activities in primary neuronal cell cultures by a SorLA antagonist/agonist

Primary neuronal cultures are prepared from brains of wild-type mice.
At postnatal day 0-2 brains are dissected out, cells are dissociated and plated in the presence of for example 10 ng/ml GDNF, and in the presence or absence of a SorLA antagonist or agonist. Following maturation of the cultures of 1-2 weeks in vitro, the number of surviving neurons was scored.

### Example 13: Modulation of GDNF activity in primary dopaminergic neurons by a SorLA antagonist/agonist

Primary dopaminergic neurons prepared from the ventral tegmental area of P0-P2 rats. The rat pups were decapitated, the brain isolated and put into cold PBS. The brain was placed ventral side down. The initial cut was through the entire brain caudal to the midbrain flexure and the second cut rostral to the flexure, the slice was laid flat. The ventral edge of the slice was cut along the top of the hypothalamus, the next cut was approximately halfway between the ventral edge of the slice and the ventricle hole. The tissue was cut into smaller segments and placed in cold L15 media (Gibco, Invitrogen). The tissue was incubated with warm papain solution (L15 media, 2 mM EDTA, 20 units/ml papain (TMWorthington, Medinova), 0.5mM kynurenic acid and NaOH (to adjust pH to approximately 7) for 30 min. at 37°C. Media was removed and tissue was disintegrated in neuron media (NeurobasalTM media without L-glutamine (Gibco, Invitrogen), FBS, B-27 (Invitrogen), glutamax (Invitrogen), primocin (Amaxa), 5-fluorodeoxyuridine (Sigma) and Uridine (Sigma)) by gently trituration. Neurons were spun down (800 rpm, 5 min) and the pellet was diluted in neuron media and plated on a layer of cortical glia cells in the presence of 10 ng/ml GDNF and in the presence or absence of a SorLA antagonist or agonist. Neurons were incubated at 37°C for 1 week in vitro whereafter they were fixed with 4% paraformaldehyde (PFA) in PBS for 30 min at room temperature. Thereafter they were washed twice for 15 min in PBS containing 0.1% Triton X-100, followed by incubation with 10% FBS in PBS for 20 min. Coverslips were hereafter incubated with anti-tyrosine hydroxylase antibodies (Pel freeze, 1:1000) in 10% FBS in PBS overnight at 4°C. Next day, the coverslips were washed three times in PBS containing 0.1% Triton X-100 and incubated with Alexa-fluor® 488 goat anti-rabbit IgG (Invitrogen, 1:1000) in 10% FBS in PBS overnight at 4°C. The coverslips were thereafter washed 2 x 15 min in 0.1 % Triton X-100 in PBS, 1 x 15 min in PBS and 1 x 15 min in water. The coverslips were mounted onto slides with Dako Flurescent Mounting Medium (Dako, Denmark). The number of surviving neurons was scored by counting the number of tyrosine hydroxylase positive neurons (as shown in Fig. 12).

### Example 14: The effect of a SorLA antagonist/agonist on amphetamine-induced hyperactivity

Mice were pretreated for 1-30 days with a SorLA antagonist or agonist, and tested for amphetamine-induced hyperactivity in an open field test consisting of a (40 x 40 x 35 cm) clear Plexiglas arena. The arena was set up in a dim room under a video camera connected to a computer under the control of the Any-maze tracking system. Mice were placed in the corner of the arena and their activity was recorded over a 40 min session as shown in Figure 13. Amphetamine (0.1 -10 mg/kg) was administered intraperitoneally or intravenously.

### Example 15: The effect of a SorLA antagonist/agonist on anxiety-related behavior

The behavior of wild-type and SorLA transgenic mice were tested for anxiety related behavior in an elevated plus maze. The elevated plus maze is used as an experimental model for depressive, manic, and anxiety-related behavior. Treatment of mice with antidepressive or anxiolytic agents normally increase the distance traveled, the number of line crossings, and the number of entries and time spent in the open arms. The elevated plus maze was raised 40 cm above the floor, and consisted of two opposite enclosed arms with 15 cm high opaque walls and two opposite open arms of the same size (35 x 5 cm). The elevated plus maze was set up in a dim lit room under a video camera connected to a computer under the control of the Any-maze tracking system. Testing sessions of 10 min were carried out for each mouse and measured the number of entries and the time spent in the open arms as described in Fig. 14. A similar experiment is performed where mice were pretreated for 1-30 days with a SorLA antagonist or agonist.

### Example 16: The effect of a SorLA antagonist/agonist on amphetamine-induced sensitization

Adult mice were pretreated with 4 mg/kg amphetamine or saline, once every other day for 5 days while also being treated with a SorLA antagonist or agonist, and tested for locomotor activity in an open field. Treatment with SorLA antagonist/agonist continued for one week after the last amphetamine pretreatment. On day 16, a test for sensitization was conducted wherein all mice received a challenge injection of amphetamine (2 mg/kg) and tested for locomotor activity in the open field.

### Example 17: The protective effect of a SorLA antagonist/agonist for MPTP-induced neurotoxicity in mice

Adult mice were injected with 20 mg/kg MPTP (1-methyl-4-phenyl-1,2,3,6-tetrahydropyridine, a agent selectively toxic to dopaminergic neurons) daily for 4 days with or without a SorLA antagonist/agonist. Motor activity was recorded, 1 day prior to injections and two days after cessation of injections in an open field test consisting of a (40 x 40 x 35 cm) clear Plexiglas arena. The arena was set up in a dim room under a video camera connected to a computer under the control of the Any-maze tracking system. Mice were placed in the corner of the arena and their activity was recorded over a 60 min session. Finally, mice were perfused with 4% paraformaldehyde for assessment of various morphological markers such as tyrosine hydroxylase and dopamine transporter using immunohistochemistry.

### Example 18: A cell based screening method for identifying agents capable of increasing cell differentiation

Neuroblastoma cells were harvested with trypsin-EDTA treatment. Cells were resuspended in DMEM-F12 media containing 10% FBS and 1% P/S (penicillin and streptomycin) and seeded in 6 well plate. The following day, the media was removed and the cells were incubated with fresh media and increasing concentrations of GDNF in the absence or presence of a SorLA antagonist or agonist. The cells were incubated for 72 hours in their normal incubator, where after the cells were visualized with a stereo-microscope (Leica DC300) which were connected to a camera. The neurite outgrowths were scored by defining a neurite as a process twice as long as the diameter of the cell.

### Example 19: Methods of treatment

The resulting developed active agent of peptide/polypeptide nature (possible antibody based) either freeze-dried to be dissolved before use or as a ready to use solution so that it can be given for parenteral administration route (e.g. intravenously (I.V.), intramuscularly (I.M.) or subcutaneously (S.C.). Mucosal application of a solid dose form also represents a possibility in this case. If the resulting developed active agent is of chemical nature a formulation for oral administration as well as a potential route is prepared e.g. for S.C. or I.M. use. The developed medicament will either be used for prophylactic purpose or given chronically for long life treatment. In this case the active agent interferes with and thereby prevents the process of molecular events to take place that leads to the symptoms of the mental and behavioural disorders. In case at that time a genetic test is developed to diagnose individuals predisposed to develop mental and behavioural disorders the medicament should be used were possible in connection with such a diagnostics. Have a mental and behavioural disorder developed; chronic treatment with the medicament represents another possibility. The rationale is constantly to be able to suppress the molecular events leading to the symptoms of the disease. Finally it will be possible to co-administer the medicament together with conventional treatments for neurological or mental and behavioural disorders e.g. with antipsychotics, antidepressants or lithium.

### Example 20: A cell based screening method for identifying agents capable of modulating internalization and degradation of a GDNF family ligand (GFL) by SorLA.

GDNF or another GFL was incubated for between 2-120 min in the presence or absence of a SorLA agonist/antagonist (e.g. an antibody) with cells expressing both SorLA and GFRα receptor. GDNF or other GFLs were labeled either by a fluorescent tag or fluorescent antibodies. The amount of internalized GDNF or GFL was subsequently evaluated using confocal microscopy as shown in Fig. 6 and Fig. 11 and Fig. 21.

### Sequence listings

**SEQ ID NO 1: Homo Sapiens SorLA polypeptide**
**SEQ ID NO 2: Homo Sapiens SorLA propetide**
   EVWTQRLHGGSAPLPQDRGFLVVQGDPRELRLWARGDARGASRADEKPLRRKR
SEQ **ID** NO 3: Homo Sapiens SorLA **Vps10p-domain**
**SEQ ID NO 4: Homo Sapiens GFRα1 polypeptide isoform a**
**SEQ ID NO 5: Homo Sapiens GFRα1 polypeptide isoform a, theoretically binding site to SorLA**
**SEQ ID NO 6: Homo Sapiens GFRα1 polypeptide isoform b**
**SEQ ID NO 7: Homo Sapiens GFRα1 polypeptide isoform b, theoretically binding site to SorLA**
**SEQ ID NO 8: Homo Sapiens GFRα2 polypeptide**
**SEQ ID NO 9: Homo Sapiens GFRα2 polypeptide, short isoform**
**SEQ ID NO 10: Homo Sapiens GFRα3 polypeptide**
**SEQ ID NO 11: Homo Sapiens GFRα3 polypeptide, isoform 2**
**SEQ ID NO 12: Homo Sapiens GFRα4 polypeptide**
**SEQ ID NO 13: Homo Sapiens Neurotensin polypeptide**
   QLYENKPRRPYIL
**SEQ ID NO 14: Homo Sapiens Neurotensin polypeptide theoretically binding site**
   YIL
**SEQ ID NO 15: Homo Sapiens Neurotensin polypeptide theoretically binding site**
   PYIL
**SEQ ID NO 16: Homo Sapiens GDNF polypeptide**
**SEQ ID NO 17: Homo Sapiens GDNF polypeptide, propeptide**
   FPLPAGKRPPEAPAEDRSLGRRRAPFALSSDSNMPEDYPDQFDDVMDFIQATIKRLKR
**SEQ ID NO 18: Homo Sapiens GDNF polypeptide, bindingsite**
   SPDKQMAVLPRRERNRQAAAANPENSRGKGRRGQRGKN

## Claims

1. An antibody binding to one or more binding sites on the extracellular domain of SorLA and increasing the extracellular levels of GDNF in the brain of a patient by inhibiting the interaction between SorLA and GFRα1for use in treating attention deficit and hyperactivity disorder (ADHD), drug abuse and anxiety disorder.

2. The antibody according to claim 1, wherein the binding site comprise SEQ ID NO: 3, or has at least 80%, such as at least 85%, 90%, 95% or 98% sequence identity to SEQ ID NO: 3.

3. The antibody according to claim 1, wherein the antibody is polyclonal or monoclonal.

4. The antibody according to claim 1, wherein the antibody is isolated or recombinant.

5. The antibody according to claim 1, wherein the antibody is a humanized, chimeric, or single-chain antibody.

## Patentansprüche

1. Antikörper, der an eine oder mehrere Bindestellen an der extrazellulären Domäne von SorLA bindet und der die extrazellulären Gehalte von GDNF in dem Gehirn eines Patienten erhöht durch Inhibieren der Interaktion zwischen SorLA und GFRα1 zur Verwendung bei der Behandlung von Aufmerksamkeitsdefizit und Hyperaktivitätsstörung (ADHD), Medikamentenmissbrauch und Angststörung.

2. Antikörper gemäß Anspruch 1, wobei die Bindungsstelle SEQ ID NR: 3 aufweist oder mindestens 80 %, wie etwa mindestens 85%, 90%, 95% oder 98%, Sequenzidentität zu der SEQ ID NR: 3 hat.

3. Antikörper gemäß Anspruch 1, wobei der Antikörper polyklonal oder monoklonal ist.

4. Antikörper gemäß Anspruch 1, wobei der Antikörper isoliert oder rekombinant ist.

5. Antikörper gemäß Anspruch 1, wobei der Antikörper ein humanisierter, chimärer oder einkettiger Antikörper ist.

## Revendications

1. Anticorps se liant à un ou plus de sites de liaison sur le domaine extracellulaire du SorLa et augmentant les niveaux extracellulaires du GDNF dans le cerveau d'un patient en inhibant l'interaction entre le SorLa et le GFRα1 pour utilisation dans le traitement d'un trouble du déficit d'attention et de l'hyperactivité (ADHB), de l'usage de drogues et d'un trouble de l'anxiété.

2. Anticorps selon la revendication 1, dans lequel le site de liaison comprend la SEQ ID NO:3 ou présente une identité de séquence d'au moins 80 %, notamment au moins 85 %, 90 %, 95 % ou 98 % avec la SEQ ID NO:3.

3. Anticorps selon la revendication 1, dans lequel l'anticorps et polyclonal ou monoclonal.

4. Anticorps selon la revendication 1, dans lequel l'anticorps est isolé ou recombinant.

5. Anticorps selon la revendication 1, dans lequel l'anticorps est un anticorps humanisé, chimérique ou à chaîne unique.


### SEQUENCE LISTING

<110> Lundbeck A/S
<120> Modulation of the interaction between SorLA and GDNF-family ligand receptors
<130> 0760
<160> 18
<170> PatentIn version 3.5
<210> 1
   <211> 2214
   <212> PRT
   <213> Homo Sapiens
<400> 1
   Met Ala Thr Arg Ser Ser Arg Arg Glu Ser Arg Leu Pro Phe Leu Phe
   1               5                   10                  15
   Thr Leu Val Ala Leu Leu Pro Pro Gly Ala Leu Cys Glu Val Trp Thr
               20                  25                  30
   Gln Arg Leu His Gly Gly Ser Ala Pro Leu Pro Gln Asp Arg Gly Phe
           35                  40                  45
   Leu Val Val Gln Gly Asp Pro Arg Glu Leu Arg Leu Trp Ala Arg Gly
       50                  55                  60
   Asp Ala Arg Gly Ala Ser Arg Ala Asp Glu Lys Pro Leu Arg Arg Lys
   65                  70                  75                  80
   Arg Ser Ala Ala Leu Gln Pro Glu Pro Ile Lys Val Tyr Gly Gln Val
                   85                  90                  95
   Ser Leu Asn Asp Ser His Asn Gln Met Val Val His Trp Ala Gly Glu
               100                 105                 110
   Lys Ser Asn Val Ile Val Ala Leu Ala Arg Asp Ser Leu Ala Leu Ala
           115                 120                 125
   Arg Pro Lys Ser Ser Asp Val Tyr Val Ser Tyr Asp Tyr Gly Lys Ser
       130                 135                 140
   Phe Lys Lys Ile Ser Asp Lys Leu Asn Phe Gly Leu Gly Asn Arg Ser
   145                 150                 155                 160
   Glu Ala Val Ile Ala Gln Phe Tyr His Ser Pro Ala Asp Asn Lys Arg
                   165                 170                 175
   Tyr Ile Phe Ala Asp Ala Tyr Ala Gln Tyr Leu Trp Ile Thr Phe Asp
   180 185 190
   Phe Cys Asn Thr Leu Gln Gly Phe Ser Ile Pro Phe Arg Ala Ala Asp
   195 200 205
   Leu Leu Leu His Ser Lys Ala Ser Asn Leu Leu Leu Gly Phe Asp Arg
   210 215 220
   Ser His Pro Asn Lys Gln Leu Trp Lys Ser Asp Asp Phe Gly Gln Thr
   225 230 235 240
   Trp Ile Met Ile Gln Glu His Val Lys Ser Phe Ser Trp Gly Ile Asp
   245 250 255
   Pro Tyr Asp Lys Pro Asn Thr Ile Tyr Ile Glu Arg His Glu Pro Ser
   260 265 270
   Gly Tyr Ser Thr Val Phe Arg Ser Thr Asp Phe Phe Gln Ser Arg Glu
   275 280 285
   Asn Gln Glu Val Ile Leu Glu Glu Val Arg Asp Phe Gln Leu Arg Asp
   290 295 300
   Lys Tyr Met Phe Ala Thr Lys Val Val His Leu Leu Gly Ser Glu Gln
   305 310 315 320
   Gln Ser Ser Val Gln Leu Trp Val Ser Phe Gly Arg Lys Pro Met Arg
   325 330 335
   Ala Ala Gln Phe Val Thr Arg His Pro Ile Asn Glu Tyr Tyr Ile Ala
   340 345 350
   Asp Ala Ser Glu Asp Gln Val Phe Val Cys Val Ser His Ser Asn Asn
   355 360 365
   Arg Thr Asn Leu Tyr Ile Ser Glu Ala Glu Gly Leu Lys Phe Ser Leu
   370 375 380
   Ser Leu Glu Asn Val Leu Tyr Tyr Ser Pro Gly Gly Ala Gly Ser Asp
   385 390 395 400
   Thr Leu Val Arg Tyr Phe Ala Asn Glu Pro Phe Ala Asp Phe His Arg
   405 410 415
   Val Glu Gly Leu Gln Gly Val Tyr Ile Ala Thr Leu Ile Asn Gly Ser
   420 425 430
   Met Asn Glu Glu Asn Met Arg Ser Val Ile Thr Phe Asp Lys Gly Gly
   435 440 445
   Thr Trp Glu Phe Leu Gln Ala Pro Ala Phe Thr Gly Tyr Gly Glu Lys
       450                 455                 460
   Ile Asn Cys Glu Leu Ser Gln Gly Cys Ser Leu His Leu Ala Gln Arg
   465                 470                 475                 480
   Leu Ser Gln Leu Leu Asn Leu Gln Leu Arg Arg Met Pro Ile Leu Ser
                   485                 490                 495
   Lys Glu Ser Ala Pro Gly Leu Ile Ile Ala Thr Gly Ser Val Gly Lys
               500                 505                 510
   Asn Leu Ala Ser Lys Thr Asn Val Tyr Ile Ser Ser Ser Ala Gly Ala
           515                 520                 525
   Arg Trp Arg Glu Ala Leu Pro Gly Pro His Tyr Tyr Thr Trp Gly Asp
       530                 535                 540
   His Gly Gly Ile Ile Thr Ala Ile Ala Gln Gly Met Glu Thr Asn Glu
   545                 550                 555                 560
   Leu Lys Tyr Ser Thr Asn Glu Gly Glu Thr Trp Lys Thr Phe Ile Phe
                   565                 570                 575
   Ser Glu Lys Pro Val Phe Val Tyr Gly Leu Leu Thr Glu Pro Gly Glu
               580                 585                 590
   Lys Ser Thr Val Phe Thr Ile Phe Gly Ser Asn Lys Glu Asn Val His
           595                 600                 605
   Ser Trp Leu Ile Leu Gln Val Asn Ala Thr Asp Ala Leu Gly Val Pro
       610                 615                 620
   Cys Thr Glu Asn Asp Tyr Lys Leu Trp Ser Pro Ser Asp Glu Arg Gly
   625                 630                 635                 640
   Asn Glu Cys Leu Leu Gly His Lys Thr Val Phe Lys Arg Arg Thr Pro
                   645                 650                 655
   His Ala Thr Cys Phe Asn Gly Glu Asp Phe Asp Arg Pro Val Val Val
               660                 665                 670
   Ser Asn Cys Ser Cys Thr Arg Glu Asp Tyr Glu Cys Asp Phe Gly Phe
           675                 680                 685
   Lys Met Ser Glu Asp Leu Ser Leu Glu Val Cys Val Pro Asp Pro Glu
       690                 695                 700
   Phe Ser Gly Lys Ser Tyr Ser Pro Pro Val Pro Cys Pro Val Gly Ser
   705                 710                 715                 720
   Thr Tyr Arg Arg Thr Arg Gly Tyr Arg Lys Ile Ser Gly Asp Thr Cys
                   725                 730                 735
   Ser Gly Gly Asp Val Glu Ala Arg Leu Glu Gly Glu Leu Val Pro Cys
               740                 745                 750
   Pro Leu Ala Glu Glu Asn Glu Phe Ile Leu Tyr Ala Val Arg Lys Ser
           755                 760                 765
   Ile Tyr Arg Tyr Asp Leu Ala Ser Gly Ala Thr Glu Gln Leu Pro Leu
       770                 775                 780
   Thr Gly Leu Arg Ala Ala Val Ala Leu Asp Phe Asp Tyr Glu His Asn
   785                 790                 795                 800
   Cys Leu Tyr Trp Ser Asp Leu Ala Leu Asp Val Ile Gln Arg Leu Cys
                   805                 810                 815
   Leu Asn Gly Ser Thr Gly Gln Glu Val Ile Ile Asn Ser Gly Leu Glu
               820                 825                 830
   Thr Val Glu Ala Leu Ala Phe Glu Pro Leu Ser Gln Leu Leu Tyr Trp
           835                 840                 845
   Val Asp Ala Gly Phe Lys Lys Ile Glu Val Ala Asn Pro Asp Gly Asp
       850                 855                 860
   Phe Arg Leu Thr Ile Val Asn Ser Ser Val Leu Asp Arg Pro Arg Ala
   865                 870                 875                 880
   Leu Val Leu Val Pro Gln Glu Gly Val Met Phe Trp Thr Asp Trp Gly
                   885                 890                 895
   Asp Leu Lys Pro Gly Ile Tyr Arg Ser Asn Met Asp Gly Ser Ala Ala
               900                 905                 910
   Tyr His Leu Val Ser Glu Asp Val Lys Trp Pro Asn Gly Ile Ser Val
           915                 920                 925
   Asp Asp Gln Trp Ile Tyr Trp Thr Asp Ala Tyr Leu Glu Cys Ile Glu
       930                 935                 940
   Arg Ile Thr Phe Ser Gly Gln Gln Arg Ser Val Ile Leu Asp Asn Leu
   945                 950                 955                 960
   Pro His Pro Tyr Ala Ile Ala Val Phe Lys Asn Glu Ile Tyr Trp Asp
                   965                 970                 975
   Asp Trp Ser Gln Leu Ser Ile Phe Arg Ala Ser Lys Tyr Ser Gly Ser
               980                 985                 990
   Gln Met Glu Ile Leu Ala Asn Gln  Leu Thr Gly Leu Met  Asp Met Lys
           995                 1000                1005
   Ile Phe  Tyr Lys Gly Lys Asn  Thr Gly Ser Asn Ala  Cys Val Pro
       1010                 1015                 1020
   Arg Pro  Cys Ser Leu Leu Cys  Leu Pro Lys Ala Asn  Asn Ser Arg
       1025                 1030                 1035
   Ser Cys  Arg Cys Pro Glu Asp  Val Ser Ser Ser Val  Leu Pro Ser
       1040                 1045                 1050
   Gly Asp  Leu Met Cys Asp Cys  Pro Gln Gly Tyr Gln  Leu Lys Asn
       1055                 1060                 1065
   Asn Thr  Cys Val Lys Gln Glu  Asn Thr Cys Leu Arg  Asn Gln Tyr
       1070                 1075                 1080
   Arg Cys  Ser Asn Gly Asn Cys  Ile Asn Ser Ile Trp  Trp Cys Asp
       1085                 1090                 1095
   Phe Asp  Asn Asp Cys Gly Asp  Met Ser Asp Glu Arg  Asn Cys Pro
       1100                 1105                 1110
   Thr Thr  Ile Cys Asp Leu Asp  Thr Gln Phe Arg Cys  Gln Glu Ser
       1115                 1120                 1125
   Gly Thr  Cys Ile Pro Leu Ser  Tyr Lys Cys Asp Leu  Glu Asp Asp
       1130                 1135                 1140
   Cys Gly  Asp Asn Ser Asp Glu  Ser His Cys Glu Met  His Gln Cys
       1145                 1150                 1155
   Arg Ser  Asp Glu Tyr Asn Cys  Ser Ser Gly Met Cys  Ile Arg Ser
       1160                 1165                 1170
   Ser Trp  Val Cys Asp Gly Asp  Asn Asp Cys Arg Asp  Trp Ser Asp
       1175                 1180                 1185
   Glu Ala  Asn Cys Thr Ala Ile  Tyr His Thr Cys Glu  Ala Ser Asn
       1190                 1195                 1200
   Phe Gln  Cys Arg Asn Gly His  Cys Ile Pro Gln Arg  Trp Ala Cys
   1205 1210 1215
   Asp Gly Asp Thr Asp Cys Gln Asp Gly Ser Asp Glu Asp Pro Val
   1220 1225 1230
   Asn Cys Glu Lys Lys Cys Asn Gly Phe Arg Cys Pro Asn Gly Thr
   1235 1240 1245
   Cys Ile Pro Ser Ser Lys His Cys Asp Gly Leu Arg Asp Cys Ser
   1250 1255 1260
   Asp Gly Ser Asp Glu Gln His Cys Glu Pro Leu Cys Thr His Phe
   1265 1270 1275
   Met Asp Phe Val Cys Lys Asn Arg Gln Gln Cys Leu Phe His Ser
   1280 1285 1290
   Met Val Cys Asp Gly Ile Ile Gln Cys Arg Asp Gly Ser Asp Glu
   1295 1300 1305
   Asp Ala Ala Phe Ala Gly Cys Ser Gln Asp Pro Glu Phe His Lys
   1310 1315 1320
   Val Cys Asp Glu Phe Gly Phe Gln Cys Gln Asn Gly Val Cys Ile
   1325 1330 1335
   Ser Leu Ile Trp Lys Cys Asp Gly Met Asp Asp Cys Gly Asp Tyr
   1340 1345 1350
   Ser Asp Glu Ala Asn Cys Glu Asn Pro Thr Glu Ala Pro Asn Cys
   1355 1360 1365
   Ser Arg Tyr Phe Gln Phe Arg Cys Glu Asn Gly His Cys Ile Pro
   1370 1375 1380
   Asn Arg Trp Lys Cys Asp Arg Glu Asn Asp Cys Gly Asp Trp Ser
   1385 1390 1395
   Asp Glu Lys Asp Cys Gly Asp Ser His Ile Leu Pro Phe Ser Thr
   1400 1405 1410
   Pro Gly Pro Ser Thr Cys Leu Pro Asn Tyr Tyr Arg Cys Ser Ser
   1415 1420 1425
   Gly Thr Cys Val Met Asp Thr Trp Val Cys Asp Gly Tyr Arg Asp
   1430 1435 1440
   Cys Ala Asp Gly Ser Asp Glu Glu Ala Cys Pro Leu Leu Ala Asn
   1445 1450 1455
   Val Thr  Ala Ala Ser Thr Pro  Thr Gln Leu Gly Arg  Cys Asp Arg
       1460                 1465                 1470
   Phe Glu  Phe Glu Cys His Gln  Pro Lys Thr Cys Ile  Pro Asn Trp
       1475                 1480                 1485
   Lys Arg  Cys Asp Gly His Gln  Asp Cys Gln Asp Gly  Arg Asp Glu
       1490                 1495                 1500
   Ala Asn  Cys Pro Thr His Ser  Thr Leu Thr Cys Met  Ser Arg Glu
       1505                 1510                 1515
   Phe Gln  Cys Glu Asp Gly Glu  Ala Cys Ile Val Leu  Ser Glu Arg
       1520                 1525                 1530
   Cys Asp  Gly Phe Leu Asp Cys  Ser Asp Glu Ser Asp  Glu Lys Ala
       1535                 1540                 1545
   Cys Ser  Asp Glu Leu Thr Val  Tyr Lys Val Gln Asn  Leu Gln Trp
       1550                 1555                 1560
   Thr Ala  Asp Phe Ser Gly Asp  Val Thr Leu Thr Trp  Met Arg Pro
       1565                 1570                 1575
   Lys Lys  Met Pro Ser Ala Ser  Cys Val Tyr Asn Val  Tyr Tyr Arg
       1580                 1585                 1590
   Val Val  Gly Glu Ser Ile Trp  Lys Thr Leu Glu Thr  His Ser Asn
       1595                 1600                 1605
   Lys Thr  Asn Thr Val Leu Lys  Val Leu Lys Pro Asp  Thr Thr Tyr
       1610                 1615                 1620
   Gln Val  Lys Val Gln Val Gln  Cys Leu Ser Lys Ala  His Asn Thr
       1625                 1630                 1635
   Asn Asp  Phe Val Thr Leu Arg  Thr Pro Glu Gly Leu  Pro Asp Ala
       1640                 1645                 1650
   Pro Arg  Asn Leu Gln Leu Ser  Leu Pro Arg Glu Ala  Glu Gly Val
       1655                 1660                 1665
   Ile Val  Gly His Trp Ala Pro  Pro Ile His Thr His  Gly Leu Ile
       1670                 1675                 1680
   Arg Glu  Tyr Ile Val Glu Tyr  Ser Arg Ser Gly Ser  Lys Met Trp
       1685                 1690                 1695
   Ala Ser Gln Arg Ala Ala Ser Asn Phe Thr Glu Ile Lys Asn Leu
   1700 1705 1710
   Leu Val Asn Thr Leu Tyr Thr Val Arg Val Ala Ala Val Thr Ser
   1715 1720 1725
   Arg Gly Ile Gly Asn Trp Ser Asp Ser Lys Ser Ile Thr Thr Ile
   1730 1735 1740
   Lys Gly Lys Val Ile Pro Pro Pro Asp Ile His Ile Asp Ser Tyr
   1745 1750 1755
   Gly Glu Asn Tyr Leu Ser Phe Thr Leu Thr Met Glu Ser Asp Ile
   1760 1765 1770
   Lys Val Asn Gly Tyr Val Val Asn Leu Phe Trp Ala Phe Asp Thr
   1775 1780 1785
   His Lys Gln Glu Arg Arg Thr Leu Asn Phe Arg Gly Ser Ile Leu
   1790 1795 1800
   Ser His Lys Val Gly Asn Leu Thr Ala His Thr Ser Tyr Glu Ile
   1805 1810 1815
   Ser Ala Trp Ala Lys Thr Asp Leu Gly Asp Ser Pro Leu Ala Phe
   1820 1825 1830
   Glu His Val Met Thr Arg Gly Val Arg Pro Pro Ala Pro Ser Leu
   1835 1840 1845
   Lys Ala Lys Ala Ile Asn Gln Thr Ala Val Glu Cys Thr Trp Thr
   1850 1855 1860
   Gly Pro Arg Asn Val Val Tyr Gly Ile Phe Tyr Ala Thr Ser Phe
   1865 1870 1875
   Leu Asp Leu Tyr Arg Asn Pro Lys Ser Leu Thr Thr Ser Leu His
   1880 1885 1890
   Asn Lys Thr Val Ile Val Ser Lys Asp Glu Gln Tyr Leu Phe Leu
   1895 1900 1905
   Val Arg Val Val Val Pro Tyr Gln Gly Pro Ser Ser Asp Tyr Val
   1910 1915 1920
   Val Val Lys Met Ile Pro Asp Ser Arg Leu Pro Pro Arg His Leu
   1925 1930 1935
   His Val  Val His Thr Gly Lys  Thr Ser Val Val Ile  Lys Trp Glu
       1940                 1945                 1950
   Ser Pro  Tyr Asp Ser Pro Asp  Gln Asp Leu Leu Tyr  Ala Val Ala
       1955                 1960                 1965
   Val Lys  Asp Leu Ile Arg Lys  Thr Asp Arg Ser Tyr  Lys Val Lys
       1970                 1975                 1980
   Ser Arg  Asn Ser Thr Val Glu  Tyr Thr Leu Asn Lys  Leu Glu Pro
       1985                 1990                 1995
   Gly Gly  Lys Tyr His Ile Ile  Val Gln Leu Gly Asn  Met Ser Lys
       2000                 2005                 2010
   Asp Ser  Ser Ile Lys Ile Thr  Thr Val Ser Leu Ser  Ala Pro Asp
       2015                 2020                 2025
   Ala Leu  Lys Ile Ile Thr Glu  Asn Asp His Val Leu  Leu Phe Trp
       2030                 2035                 2040
   Lys Ser  Leu Ala Leu Lys Glu  Lys His Phe Asn Glu  Ser Arg Gly
       2045                 2050                 2055
   Tyr Glu  Ile His Met Phe Asp  Ser Ala Met Asn Ile  Thr Ala Tyr
       2060                 2065                 2070
   Leu Gly  Asn Thr Thr Asp Asn  Phe Phe Lys Ile Ser  Asn Leu Lys
       2075                 2080                 2085
   Met Gly  His Asn Tyr Thr Phe  Thr Val Gln Ala Arg  Cys Leu Phe
       2090                 2095                 2100
   Gly Asn  Gln Ile Cys Gly Glu  Pro Ala Ile Leu Leu  Tyr Asp Glu
       2105                 2110                 2115
   Leu Gly  Ser Gly Ala Asp Ala  Ser Ala Thr Gln Ala  Ala Arg Ser
       2120                 2125                 2130
   Thr Asp  Val Ala Ala Val Val  Val Pro Ile Leu Phe  Leu Ile Leu
       2135                 2140                 2145
   Leu Ser  Leu Gly Val Gly Phe  Ala Ile Leu Tyr Thr  Lys His Arg
       2150                 2155                 2160
   Arg Leu  Gln Ser Ser Phe Thr  Ala Phe Ala Asn Ser  His Tyr Ser
       2165                 2170                 2175
   Ser Arg  Leu Gly Ser Ala Ile  Phe Ser Ser Gly Asp  Asp Leu Gly
   2180 2185 2190
   Glu Asp Asp Glu Asp Ala Pro Met Ile Thr Gly Phe Ser Asp Asp
   2195 2200 2205
   Val Pro Met Val Ile Ala
   2210
<210> 2
   <211> 53
   <212> PRT
   <213> Homo Sapiens
<400> 2
   Glu Val Trp Thr Gln Arg Leu His Gly Gly Ser Ala Pro Leu Pro Gln
   1 5 10 15
   Asp Arg Gly Phe Leu Val Val Gln Gly Asp Pro Arg Glu Leu Arg Leu
   20 25 30
   Trp Ala Arg Gly Asp Ala Arg Gly Ala Ser Arg Ala Asp Glu Lys Pro
   35 40 45
   Leu Arg Arg Lys Arg
   50
<210> 3
   <211> 676
   <212> PRT
   <213> Homo Sapiens
<400> 3
   Ser Ala Ala Leu Gln Pro Glu Pro Ile Lys Val Tyr Gly Gln Val Ser
   1               5                   10                  15
   Leu Asn Asp Ser His Asn Gln Met Val Val His Trp Ala Gly Glu Lys
               20                  25                  30
   Ser Asn Val Ile Val Ala Leu Ala Arg Asp Ser Leu Ala Leu Ala Arg
           35                  40                  45
   Pro Lys Ser Ser Asp Val Tyr Val Ser Tyr Asp Tyr Gly Lys Ser Phe
       50                  55                  60
   Lys Lys Ile Ser Asp Lys Leu Asn Phe Gly Leu Gly Asn Arg Ser Glu
   65                  70                  75                  80
   Ala Val Ile Ala Gln Phe Tyr His Ser Pro Ala Asp Asn Lys Arg Tyr
                   85                  90                  95
   Ile Phe Ala Asp Ala Tyr Ala Gln Tyr Leu Trp Ile Thr Phe Asp Phe
               100                 105                 110
   Cys Asn Thr Leu Gln Gly Phe Ser Ile Pro Phe Arg Ala Ala Asp Leu
           115                 120                 125
   Leu Leu His Ser Lys Ala Ser Asn Leu Leu Leu Gly Phe Asp Arg Ser
       130                 135                 140
   His Pro Asn Lys Gln Leu Trp Lys Ser Asp Asp Phe Gly Gln Thr Trp
   145                 150                 155                 160
   Ile Met Ile Gln Glu His Val Lys Ser Phe Ser Trp Gly Ile Asp Pro
                   165                 170                 175
   Tyr Asp Lys Pro Asn Thr Ile Tyr Ile Glu Arg His Glu Pro Ser Gly
               180                 185                 190
   Tyr Ser Thr Val Phe Arg Ser Thr Asp Phe Phe Gln Ser Arg Glu Asn
           195                 200                 205
   Gln Glu Val Ile Leu Glu Glu Val Arg Asp Phe Gln Leu Arg Asp Lys
       210                 215                 220
   Tyr Met Phe Ala Thr Lys Val Val His Leu Leu Gly Ser Glu Gln Gln
   225                 230                 235                 240
   Ser Ser Val Gln Leu Trp Val Ser Phe Gly Arg Lys Pro Met Arg Ala
                   245                 250                 255
   Ala Gln Phe Val Thr Arg His Pro Ile Asn Glu Tyr Tyr Ile Ala Asp
               260                 265                 270
   Ala Ser Glu Asp Gln Val Phe Val Cys Val Ser His Ser Asn Asn Arg
           275                 280                 285
   Thr Asn Leu Tyr Ile Ser Glu Ala Glu Gly Leu Lys Phe Ser Leu Ser
       290                 295                 300
   Leu Glu Asn Val Leu Tyr Tyr Ser Pro Gly Gly Ala Gly Ser Asp Thr
   305                 310                 315                 320
   Leu Val Arg Tyr Phe Ala Asn Glu Pro Phe Ala Asp Phe His Arg Val
                   325                 330                 335
   Glu Gly Leu Gln Gly Val Tyr Ile Ala Thr Leu Ile Asn Gly Ser Met
               340                 345                 350
   Asn Glu Glu Asn Met Arg Ser Val Ile Thr Phe Asp Lys Gly Gly Thr
   355 360 365
   Trp Glu Phe Leu Gln Ala Pro Ala Phe Thr Gly Tyr Gly Glu Lys Ile
   370 375 380
   Asn Cys Glu Leu Ser Gln Gly Cys Ser Leu His Leu Ala Gln Arg Leu
   385 390 395 400
   Ser Gln Leu Leu Asn Leu Gln Leu Arg Arg Met Pro Ile Leu Ser Lys
   405 410 415
   Glu Ser Ala Pro Gly Leu Ile Ile Ala Thr Gly Ser Val Gly Lys Asn
   420 425 430
   Leu Ala Ser Lys Thr Asn Val Tyr Ile Ser Ser Ser Ala Gly Ala Arg
   435 440 445
   Trp Arg Glu Ala Leu Pro Gly Pro His Tyr Tyr Thr Trp Gly Asp His
   450 455 460
   Gly Gly Ile Ile Thr Ala Ile Ala Gln Gly Met Glu Thr Asn Glu Leu
   465 470 475 480
   Lys Tyr Ser Thr Asn Glu Gly Glu Thr Trp Lys Thr Phe Ile Phe Ser
   485 490 495
   Glu Lys Pro Val Phe Val Tyr Gly Leu Leu Thr Glu Pro Gly Glu Lys
   500 505 510
   Ser Thr Val Phe Thr Ile Phe Gly Ser Asn Lys Glu Asn Val His Ser
   515 520 525
   Trp Leu Ile Leu Gln Val Asn Ala Thr Asp Ala Leu Gly Val Pro Cys
   530 535 540
   Thr Glu Asn Asp Tyr Lys Leu Trp Ser Pro Ser Asp Glu Arg Gly Asn
   545 550 555 560
   Glu Cys Leu Leu Gly His Lys Thr Val Phe Lys Arg Arg Thr Pro His
   565 570 575
   Ala Thr Cys Phe Asn Gly Glu Asp Phe Asp Arg Pro Val Val Val Ser
   580 585 590
   Asn Cys Ser Cys Thr Arg Glu Asp Tyr Glu Cys Asp Phe Gly Phe Lys
   595 600 605
   Met Ser Glu Asp Leu Ser Leu Glu Val Cys Val Pro Asp Pro Glu Phe
   610 615 620
   Ser Gly Lys Ser Tyr Ser Pro Pro Val Pro Cys Pro Val Gly Ser Thr
   625 630 635 640
   Tyr Arg Arg Thr Arg Gly Tyr Arg Lys Ile Ser Gly Asp Thr Cys Ser
   645 650 655
   Gly Gly Asp Val Glu Ala Arg Leu Glu Gly Glu Leu Val Pro Cys Pro
   660 665 670
   Leu Ala Glu Glu
   675
<210> 4
   <211> 465
   <212> PRT
   <213> Homo Sapiens
<400> 4
   Met Phe Leu Ala Thr Leu Tyr Phe Ala Leu Pro Leu Leu Asp Leu Leu
   1 5 10 15
   Leu Ser Ala Glu Val Ser Gly Gly Asp Arg Leu Asp Cys Val Lys Ala
   20 25 30
   Ser Asp Gln Cys Leu Lys Glu Gln Ser Cys Ser Thr Lys Tyr Arg Thr
   35 40 45
   Leu Arg Gln Cys Val Ala Gly Lys Glu Thr Asn Phe Ser Leu Ala Ser
   50 55 60
   Gly Leu Glu Ala Lys Asp Glu Cys Arg Ser Ala Met Glu Ala Leu Lys
   65 70 75 80
   Gln Lys Ser Leu Tyr Asn Cys Arg Cys Lys Arg Gly Met Lys Lys Glu
   85 90 95
   Lys Asn Cys Leu Arg Ile Tyr Trp Ser Met Tyr Gln Ser Leu Gln Gly
   100 105 110
   Asn Asp Leu Leu Glu Asp Ser Pro Tyr Glu Pro Val Asn Ser Arg Leu
   115 120 125
   Ser Asp Ile Phe Arg Val Val Pro Phe Ile Ser Asp Val Phe Gln Gln
   130 135 140
   Val Glu His Ile Pro Lys Gly Asn Asn Cys Leu Asp Ala Ala Lys Ala
   145 150 155 160
   Cys Asn Leu Asp Asp Ile Cys Lys Lys Tyr Arg Ser Ala Tyr Ile Thr
                   165                 170                 175
   Pro Cys Thr Thr Ser Val Ser Asn Asp Val Cys Asn Arg Arg Lys Cys
               180                 185                 190
   His Lys Ala Leu Arg Gln Phe Phe Asp Lys Val Pro Ala Lys His Ser
           195                 200                 205
   Tyr Gly Met Leu Phe Cys Ser Cys Arg Asp Ile Ala Cys Thr Glu Arg
       210                 215                 220
   Arg Arg Gln Thr Ile Val Pro Val Cys Ser Tyr Glu Glu Arg Glu Lys
   225                 230                 235                 240
   Pro Asn Cys Leu Asn Leu Gln Asp Ser Cys Lys Thr Asn Tyr Ile Cys
                   245                 250                 255
   Arg Ser Arg Leu Ala Asp Phe Phe Thr Asn Cys Gln Pro Glu Ser Arg
               260                 265                 270
   Ser Val Ser Ser Cys Leu Lys Glu Asn Tyr Ala Asp Cys Leu Leu Ala
           275                 280                 285
   Tyr Ser Gly Leu Ile Gly Thr Val Met Thr Pro Asn Tyr Ile Asp Ser
       290                 295                 300
   Ser Ser Leu Ser Val Ala Pro Trp Cys Asp Cys Ser Asn Ser Gly Asn
   305                 310                 315                 320
   Asp Leu Glu Glu Cys Leu Lys Phe Leu Asn Phe Phe Lys Asp Asn Thr
                   325                 330                 335
   Cys Leu Lys Asn Ala Ile Gln Ala Phe Gly Asn Gly Ser Asp Val Thr
               340                 345                 350
   Val Trp Gln Pro Ala Phe Pro Val Gln Thr Thr Thr Ala Thr Thr Thr
           355                 360                 365
   Thr Ala Leu Arg Val Lys Asn Lys Pro Leu Gly Pro Ala Gly Ser Glu
       370                 375                 380
   Asn Glu Ile Pro Thr His Val Leu Pro Pro Cys Ala Asn Leu Gln Ala
   385                 390                 395                 400
   Gln Lys Leu Lys Ser Asn Val Ser Gly Asn Thr His Leu Cys Ile Ser
                   405                 410                 415
   Asn Gly Asn Tyr Glu Lys Glu Gly Leu Gly Ala Ser Ser His Ile Thr
                   420                 425                 430
   Thr Lys Ser Met Ala Ala Pro Pro Ser Cys Gly Leu Ser Pro Leu Leu
           435                 440                 445
   Val Leu Val Val Thr Ala Leu Ser Thr Leu Leu Ser Leu Thr Glu Thr
       450                 455                 460
   Ser
   465
<210> 5
   <211> 120
   <212> PRT
   <213> Homo Sapiens
<400> 5
   Asp Arg Leu Asp Cys Val Lys Ala Ser Asp Gln Cys Leu Lys Glu Gln
   1               5                   10                  15
   Ser Cys Ser Thr Lys Tyr Arg Thr Leu Arg Gln Cys Val Ala Gly Lys
               20                  25                  30
   Glu Thr Asn Phe Ser Leu Ala Ser Gly Leu Glu Ala Lys Asp Glu Cys
           35                  40                  45
   Arg Ser Ala Met Glu Ala Leu Lys Gln Lys Ser Leu Tyr Asn Cys Arg
       50                  55                  60
   Cys Lys Arg Gly Met Lys Lys Glu Lys Asn Cys Leu Arg Ile Tyr Trp
   65                  70                  75                  80
   Ser Met Tyr Gln Ser Leu Gln Gly Asn Asp Leu Leu Glu Asp Ser Pro
                   85                  90                  95
   Tyr Glu Pro Val Asn Ser Arg Leu Ser Asp Ile Phe Arg Val Val Pro
               100                 105                 110
   Phe Ile Ser Asp Val Phe Gln Gln
           115                 120
<210> 6
   <211> 460
   <212> PRT
   <213> Homo Sapiens
<400> 6
   Met Phe Leu Ala Thr Leu Tyr Phe Ala Leu Pro Leu Leu Asp Leu Leu
   1               5                   10                  15
   Leu Ser Ala Glu Val Ser Gly Gly Asp Arg Leu Asp Cys Val Lys Ala
               20                  25                  30
   Ser Asp Gln Cys Leu Lys Glu Gln Ser Cys Ser Thr Lys Tyr Arg Thr
           35                  40                  45
   Leu Arg Gln Cys Val Ala Gly Lys Glu Thr Asn Phe Ser Leu Ala Ser
       50                  55                  60
   Gly Leu Glu Ala Lys Asp Glu Cys Arg Ser Ala Met Glu Ala Leu Lys
   65                  70                  75                  80
   Gln Lys Ser Leu Tyr Asn Cys Arg Cys Lys Arg Gly Met Lys Lys Glu
                   85                  90                  95
   Lys Asn Cys Leu Arg Ile Tyr Trp Ser Met Tyr Gln Ser Leu Gln Gly
               100                 105                 110
   Asn Asp Leu Leu Glu Asp Ser Pro Tyr Glu Pro Val Asn Ser Arg Leu
           115                 120                 125
   Ser Asp Ile Phe Arg Val Val Pro Phe Ile Ser Val Glu His Ile Pro
       130                 135                 140
   Lys Gly Asn Asn Cys Leu Asp Ala Ala Lys Ala Cys Asn Leu Asp Asp
   145                 150                 155                 160
   Ile Cys Lys Lys Tyr Arg Ser Ala Tyr Ile Thr Pro Cys Thr Thr Ser
                   165                 170                 175
   Val Ser Asn Asp Val Cys Asn Arg Arg Lys Cys His Lys Ala Leu Arg
               180                 185                 190
   Gln Phe Phe Asp Lys Val Pro Ala Lys His Ser Tyr Gly Met Leu Phe
           195                 200                 205
   Cys Ser Cys Arg Asp Ile Ala Cys Thr Glu Arg Arg Arg Gln Thr Ile
       210                 215                 220
   Val Pro Val Cys Ser Tyr Glu Glu Arg Glu Lys Pro Asn Cys Leu Asn
   225                 230                 235                 240
   Leu Gln Asp Ser Cys Lys Thr Asn Tyr Ile Cys Arg Ser Arg Leu Ala
                   245                 250                 255
   Asp Phe Phe Thr Asn Cys Gln Pro Glu Ser Arg Ser Val Ser Ser Cys
               260                 265                 270
   Leu Lys Glu Asn Tyr Ala Asp Cys Leu Leu Ala Tyr Ser Gly Leu Ile
               275                 280                 285
   Gly Thr Val Met Thr Pro Asn Tyr Ile Asp Ser Ser Ser Leu Ser Val
       290                 295                 300
   Ala Pro Trp Cys Asp Cys Ser Asn Ser Gly Asn Asp Leu Glu Glu Cys
   305                 310                 315                 320
   Leu Lys Phe Leu Asn Phe Phe Lys Asp Asn Thr Cys Leu Lys Asn Ala
                   325                 330                 335
   Ile Gln Ala Phe Gly Asn Gly Ser Asp Val Thr Val Trp Gln Pro Ala
               340                 345                 350
   Phe Pro Val Gln Thr Thr Thr Ala Thr Thr Thr Thr Ala Leu Arg Val
           355                 360                 365
   Lys Asn Lys Pro Leu Gly Pro Ala Gly Ser Glu Asn Glu Ile Pro Thr
       370                 375                 380
   His Val Leu Pro Pro Cys Ala Asn Leu Gln Ala Gln Lys Leu Lys Ser
   385                 390                 395                 400
   Asn Val Ser Gly Asn Thr His Leu Cys Ile Ser Asn Gly Asn Tyr Glu
                   405                 410                 415
   Lys Glu Gly Leu Gly Ala Ser Ser His Ile Thr Thr Lys Ser Met Ala
               420                 425                 430
   Ala Pro Pro Ser Cys Gly Leu Ser Pro Leu Leu Val Leu Val Val Thr
           435                 440                 445
   Ala Leu Ser Thr Leu Leu Ser Leu Thr Glu Thr Ser
       450                 455                 460
<210> 7
   <211> 115
   <212> PRT
   <213> Homo Sapiens
<400> 7
   Asp Arg Leu Asp Cys Val Lys Ala Ser Asp Gln Cys Leu Lys Glu Gln
   1               5                   10                  15
   Ser Cys Ser Thr Lys Tyr Arg Thr Leu Arg Gln Cys Val Ala Gly Lys
               20                  25                  30
   Glu Thr Asn Phe Ser Leu Ala Ser Gly Leu Glu Ala Lys Asp Glu Cys
           35                  40                  45
   Arg Ser Ala Met Glu Ala Leu Lys Gln Lys Ser Leu Tyr Asn Cys Arg
       50                  55                  60
   Cys Lys Arg Gly Met Lys Lys Glu Lys Asn Cys Leu Arg Ile Tyr Trp
   65                  70                  75                  80
   Ser Met Tyr Gln Ser Leu Gln Gly Asn Asp Leu Leu Glu Asp Ser Pro
                   85                  90                  95
   Tyr Glu Pro Val Asn Ser Arg Leu Ser Asp Ile Phe Arg Val Val Pro
               100                 105                 110
   Phe Ile Ser
           115
<210> 8
   <211> 464
   <212> PRT
   <213> Homo Sapiens
<400> 8
   Met Ile Leu Ala Asn Val Phe Cys Leu Phe Phe Phe Leu Asp Glu Thr
   1               5                   10                  15
   Leu Arg Ser Leu Ala Ser Pro Ser Ser Leu Gln Gly Pro Glu Leu His
               20                  25                  30
   Gly Trp Arg Pro Pro Val Asp Cys Val Arg Ala Asn Glu Leu Cys Ala
           35                  40                  45
   Ala Glu Ser Asn Cys Ser Ser Arg Tyr Arg Thr Leu Arg Gln Cys Leu
       50                  55                  60
   Ala Gly Arg Asp Arg Asn Thr Met Leu Ala Asn Lys Glu Cys Gln Ala
   65                  70                  75                  80
   Ala Leu Glu Val Leu Gln Glu Ser Pro Leu Tyr Asp Cys Arg Cys Lys
                   85                  90                  95
   Arg Gly Met Lys Lys Glu Leu Gln Cys Leu Gln Ile Tyr Trp Ser Ile
               100                 105                 110
   His Leu Gly Leu Thr Glu Gly Glu Glu Phe Tyr Glu Ala Ser Pro Tyr
           115                 120                 125
   Glu Pro Val Thr Ser Arg Leu Ser Asp Ile Phe Arg Leu Ala Ser Ile
       130                 135                 140
   Phe Ser Gly Thr Gly Ala Asp Pro Val Val Ser Ala Lys Ser Asn His
   145                 150                 155                 160
   Cys Leu Asp Ala Ala Lys Ala Cys Asn Leu Asn Asp Asn Cys Lys Lys
                   165                 170                 175
   Leu Arg Ser Ser Tyr Ile Ser Ile Cys Asn Arg Glu Ile Ser Pro Thr
               180                 185                 190
   Glu Arg Cys Asn Arg Arg Lys Cys His Lys Ala Leu Arg Gln Phe Phe
           195                 200                 205
   Asp Arg Val Pro Ser Glu Tyr Thr Tyr Arg Met Leu Phe Cys Ser Cys
       210                 215                 220
   Gln Asp Gln Ala Cys Ala Glu Arg Arg Arg Gln Thr Ile Leu Pro Ser
   225                 230                 235                 240
   Cys Ser Tyr Glu Asp Lys Glu Lys Pro Asn Cys Leu Asp Leu Arg Gly
                   245                 250                 255
   Val Cys Arg Thr Asp His Leu Cys Arg Ser Arg Leu Ala Asp Phe His
               260                 265                 270
   Ala Asn Cys Arg Ala Ser Tyr Gln Thr Val Thr Ser Cys Pro Ala Asp
           275                 280                 285
   Asn Tyr Gln Ala Cys Leu Gly Ser Tyr Ala Gly Met Ile Gly Phe Asp
       290                 295                 300
   Met Thr Pro Asn Tyr Val Asp Ser Ser Pro Thr Gly Ile Val Val Ser
   305                 310                 315                 320
   Pro Trp Cys Ser Cys Arg Gly Ser Gly Asn Met Glu Glu Glu Cys Glu
                   325                 330                 335
   Lys Phe Leu Arg Asp Phe Thr Glu Asn Pro Cys Leu Arg Asn Ala Ile
               340                 345                 350
   Gln Ala Phe Gly Asn Gly Thr Asp Val Asn Val Ser Pro Lys Gly Pro
           355                 360                 365
   Ser Phe Gln Ala Thr Gln Ala Pro Arg Val Glu Lys Thr Pro Ser Leu
       370                 375                 380
   Pro Asp Asp Leu Ser Asp Ser Thr Ser Leu Gly Thr Ser Val Ile Thr
   385                 390                 395                 400
   Thr Cys Thr Ser Val Gln Glu Gln Gly Leu Lys Ala Asn Asn Ser Lys
                   405                 410                 415
   Glu Leu Ser Met Cys Phe Thr Glu Leu Thr Thr Asn Ile Ile Pro Gly
               420                 425                 430
   Ser Asn Lys Val Ile Lys Pro Asn Ser Gly Pro Ser Arg Ala Arg Pro
           435                 440                 445
   Ser Ala Ala Leu Thr Val Leu Ser Val Leu Met Leu Lys Leu Ala Leu
       450                 455                 460
<210> 9
   <211> 331
   <212> PRT
   <213> Homo Sapiens
<400> 9
   Met Ile Leu Ala Asn Val Phe Cys Leu Phe Phe Phe Leu Gly Thr Gly
   1               5                   10                  15
   Ala Asp Pro Val Val Ser Ala Lys Ser Asn His Cys Leu Asp Ala Ala
               20                  25                  30
   Lys Ala Cys Asn Leu Asn Asp Asn Cys Lys Lys Leu Arg Ser Ser Tyr
           35                  40                  45
   Ile Ser Ile Cys Asn Arg Glu Ile Ser Pro Thr Glu Arg Cys Asn Arg
       50                  55                  60
   Arg Lys Cys His Lys Ala Leu Arg Gln Phe Phe Asp Arg Val Pro Ser
   65                  70                  75                  80
   Glu Tyr Thr Tyr Arg Met Leu Phe Cys Ser Cys Gln Asp Gln Ala Cys
                   85                  90                  95
   Ala Glu Arg Arg Arg Gln Thr Ile Leu Pro Ser Cys Ser Tyr Glu Asp
               100                 105                 110
   Lys Glu Lys Pro Asn Cys Leu Asp Leu Arg Gly Val Cys Arg Thr Asp
           115                 120                 125
   His Leu Cys Arg Ser Arg Leu Ala Asp Phe His Ala Asn Cys Arg Ala
       130                 135                 140
   Ser Tyr Gln Thr Val Thr Ser Cys Pro Ala Asp Asn Tyr Gln Ala Cys
   145                 150                 155                 160
   Leu Gly Ser Tyr Ala Gly Met Ile Gly Phe Asp Met Thr Pro Asn Tyr
                   165                 170                 175
   Val Asp Ser Ser Pro Thr Gly Ile Val Val Ser Pro Trp Cys Ser Cys
               180                 185                 190
   Arg Gly Ser Gly Asn Met Glu Glu Glu Cys Glu Lys Phe Leu Arg Asp
           195                 200                 205
   Phe Thr Glu Asn Pro Cys Leu Arg Asn Ala Ile Gln Ala Phe Gly Asn
       210                 215                 220
   Gly Thr Asp Val Asn Val Ser Pro Lys Gly Pro Ser Phe Gln Ala Thr
   225                 230                 235                 240
   Gln Ala Pro Arg Val Glu Lys Thr Pro Ser Leu Pro Asp Asp Leu Ser
                   245                 250                 255
   Asp Ser Thr Ser Leu Gly Thr Ser Val Ile Thr Thr Cys Thr Ser Val
               260                 265                 270
   Gln Glu Gln Gly Leu Lys Ala Asn Asn Ser Lys Glu Leu Ser Met Cys
           275                 280                 285
   Phe Thr Glu Leu Thr Thr Asn Ile Ile Pro Gly Ser Asn Lys Val Ile
       290                 295                 300
   Lys Pro Asn Ser Gly Pro Ser Arg Ala Arg Pro Ser Ala Ala Leu Thr
   305                 310                 315                 320
   Val Leu Ser Val Leu Met Leu Lys Leu Ala Leu
                   325                 330
<210> 10
   <211> 400
   <212> PRT
   <213> Homo Sapiens
<400> 10
   Met Val Arg Pro Leu Asn Pro Arg Pro Leu Pro Pro Val Val Leu Met
   1               5                   10                  15
   Leu Leu Leu Leu Leu Pro Pro Ser Pro Leu Pro Leu Ala Ala Gly Asp
               20                  25                  30
   Pro Leu Pro Thr Glu Ser Arg Leu Met Asn Ser Cys Leu Gln Ala Arg
           35                  40                  45
   Arg Lys Cys Gln Ala Asp Pro Thr Cys Ser Ala Ala Tyr His His Leu
       50                  55                  60
   Asp Ser Cys Thr Ser Ser Ile Ser Thr Pro Leu Pro Ser Glu Glu Pro
   65                  70                  75                  80
   Ser Val Pro Ala Asp Cys Leu Glu Ala Ala Gln Gln Leu Arg Asn Ser
                   85                  90                  95
   Ser Leu Ile Gly Cys Met Cys His Arg Arg Met Lys Asn Gln Val Ala
               100                 105                 110
   Cys Leu Asp Ile Tyr Trp Thr Val His Arg Ala Arg Ser Leu Gly Asn
           115                 120                 125
   Tyr Glu Leu Asp Val Ser Pro Tyr Glu Asp Thr Val Thr Ser Lys Pro
       130                 135                 140
   Trp Lys Met Asn Leu Ser Lys Leu Asn Met Leu Lys Pro Asp Ser Asp
   145                 150                 155                 160
   Leu Cys Leu Lys Phe Ala Met Leu Cys Thr Leu Asn Asp Lys Cys Asp
                   165                 170                 175
   Arg Leu Arg Lys Ala Tyr Gly Glu Ala Cys Ser Gly Pro His Cys Gln
               180                 185                 190
   Arg His Val Cys Leu Arg Gln Leu Leu Thr Phe Phe Glu Lys Ala Ala
           195                 200                 205
   Glu Pro His Ala Gln Gly Leu Leu Leu Cys Pro Cys Ala Pro Asn Asp
       210                 215                 220
   Arg Gly Cys Gly Glu Arg Arg Arg Asn Thr Ile Ala Pro Asn Cys Ala
   225                 230                 235                 240
   Leu Pro Pro Val Ala Pro Asn Cys Leu Glu Leu Arg Arg Leu Cys Phe
                   245                 250                 255
   Ser Asp Pro Leu Cys Arg Ser Arg Leu Val Asp Phe Gln Thr His Cys
               260                 265                 270
   His Pro Met Asp Ile Leu Gly Thr Cys Ala Thr Glu Gln Ser Arg Cys
           275                 280                 285
   Leu Arg Ala Tyr Leu Gly Leu Ile Gly Thr Ala Met Thr Pro Asn Phe
       290                 295                 300
   Val Ser Asn Val Asn Thr Ser Val Ala Leu Ser Cys Thr Cys Arg Gly
   305                 310                 315                 320
   Ser Gly Asn Leu Gln Glu Glu Cys Glu Met Leu Glu Gly Phe Phe Ser
                   325                 330                 335
   His Asn Pro Cys Leu Thr Glu Ala Ile Ala Ala Lys Met Arg Phe His
   340 345 350
   Ser Gln Leu Phe Ser Gln Asp Trp Pro His Pro Thr Phe Ala Val Met
   355 360 365
   Ala His Gln Asn Glu Asn Pro Ala Val Arg Pro Gln Pro Trp Val Pro
   370 375 380
   Ser Leu Phe Ser Cys Thr Leu Pro Leu Ile Leu Leu Leu Ser Leu Trp
   385 390 395 400
<210> 11
   <211> 369
   <212> PRT
   <213> Homo Sapiens
<400> 11
   Met Val Arg Pro Leu Asn Pro Arg Pro Leu Pro Pro Val Val Leu Met
   1               5                   10                  15
   Leu Leu Leu Leu Leu Pro Pro Ser Pro Leu Pro Leu Ala Ala Gly Asp
               20                  25                  30
   Pro Leu Pro Thr Glu Ser Arg Leu Met Asn Ser Cys Leu Gln Ala Arg
           35                  40                  45
   Arg Lys Cys Gln Ala Asp Pro Thr Cys Ser Ala Ala Tyr His His Leu
       50                  55                  60
   Asp Ser Cys Thr Ser Ser Ile Ser Thr Pro Leu Pro Ser Glu Glu Pro
   65                  70                  75                  80
   Ser Val Pro Ala Asp Cys Leu Glu Ala Ala Gln Gln Leu Arg Asn Ser
                   85                  90                  95
   Ser Leu Ile Gly Cys Met Cys His Arg Arg Met Lys Asn Gln Val Ala
               100                 105                 110
   Cys Leu Asp Ile Tyr Trp Thr Val His Arg Ala Arg Ser Leu Asp Ser
           115                 120                 125
   Asp Leu Cys Leu Lys Phe Ala Met Leu Cys Thr Leu Asn Asp Lys Cys
       130                 135                 140
   Asp Arg Leu Arg Lys Ala Tyr Gly Glu Ala Cys Ser Gly Pro His Cys
   145                 150                 155                 160
   Gln Arg His Val Cys Leu Arg Gln Leu Leu Thr Phe Phe Glu Lys Ala
                       165                 170                 175
   Ala Glu Pro His Ala Gln Gly Leu Leu Leu Cys Pro Cys Ala Pro Asn
               180                 185                 190
   Asp Arg Gly Cys Gly Glu Arg Arg Arg Asn Thr Ile Ala Pro Asn Cys
           195                 200                 205
   Ala Leu Pro Pro Val Ala Pro Asn Cys Leu Glu Leu Arg Arg Leu Cys
       210                 215                 220
   Phe Ser Asp Pro Leu Cys Arg Ser Arg Leu Val Asp Phe Gln Thr His
   225                 230                 235                 240
   Cys His Pro Met Asp Ile Leu Gly Thr Cys Ala Thr Glu Gln Ser Arg
                   245                 250                 255
   Cys Leu Arg Ala Tyr Leu Gly Leu Ile Gly Thr Ala Met Thr Pro Asn
               260                 265                 270
   Phe Val Ser Asn Val Asn Thr Ser Val Ala Leu Ser Cys Thr Cys Arg
           275                 280                 285
   Gly Ser Gly Asn Leu Gln Glu Glu Cys Glu Met Leu Glu Gly Phe Phe
       290                 295                 300
   Ser His Asn Pro Cys Leu Thr Glu Ala Ile Ala Ala Lys Met Arg Phe
   305                 310                 315                 320
   His Ser Gln Leu Phe Ser Gln Asp Trp Pro His Pro Thr Phe Ala Val
                   325                 330                 335
   Met Ala His Gln Asn Glu Asn Pro Ala Val Arg Pro Gln Pro Trp Val
               340                 345                 350
   Pro Ser Leu Phe Ser Cys Thr Leu Pro Leu Ile Leu Leu Leu Ser Leu
           355                 360                 365
   Trp
<210> 12
   <211> 299
   <212> PRT
   <213> Homo Sapiens
<400> 12
   Met Val Arg Cys Leu Gly Pro Ala Leu Leu Leu Leu Leu Leu Leu Gly
   1               5                   10                  15
   Ser Ala Ser Ser Val Gly Gly Asn Arg Cys Val Asp Ala Ala Glu Ala
               20                  25                  30
   Cys Thr Ala Asp Ala Arg Cys Gln Arg Leu Arg Ser Glu Tyr Val Ala
           35                  40                  45
   Gln Cys Leu Gly Arg Ala Ala Gln Gly Gly Cys Pro Arg Ala Arg Cys
       50                  55                  60
   Arg Arg Ala Leu Arg Arg Phe Phe Ala Arg Gly Pro Pro Ala Leu Thr
   65                  70                  75                  80
   His Ala Leu Leu Phe Cys Pro Cys Ala Gly Pro Ala Cys Ala Glu Arg
                   85                  90                  95
   Arg Arg Gln Thr Phe Val Pro Ser Cys Ala Phe Ser Gly Pro Gly Pro
               100                 105                 110
   Ala Pro Pro Ser Cys Leu Glu Pro Leu Asn Phe Cys Glu Arg Ser Arg
           115                 120                 125
   Val Cys Arg Cys Ala Arg Ala Ala Ala Gly Pro Trp Arg Gly Trp Gly
       130                 135                 140
   Arg Gly Leu Ser Pro Ala His Arg Pro Pro Ala Ala Gln Ala Ser Pro
   145                 150                 155                 160
   Pro Gly Leu Ser Gly Leu Val His Pro Ser Ala Gln Arg Pro Arg Arg
                   165                 170                 175
   Leu Pro Ala Gly Pro Gly Arg Pro Leu Pro Ala Arg Leu Arg Gly Pro
               180                 185                 190
   Arg Gly Val Pro Ala Gly Thr Ala Val Thr Pro Asn Tyr Val Asp Asn
           195                 200                 205
   Val Ser Ala Arg Val Ala Pro Trp Cys Asp Cys Gly Ala Ser Gly Asn
       210                 215                 220
   Arg Arg Glu Asp Cys Glu Ala Phe Arg Gly Leu Phe Thr Arg Asn Arg
   225                 230                 235                 240
   Cys Leu Asp Gly Ala Ile Gln Ala Phe Ala Ser Gly Trp Pro Pro Val
                   245                 250                 255
   Leu Leu Asp Gln Leu Asn Pro Gln Gly Asp Pro Glu His Ser Leu Leu
               260                 265                 270
   Gln Val Ser Ser Thr Gly Arg Ala Leu Glu Arg Arg Ser Leu Leu Ser
   275 280 285
   Ile Leu Pro Val Leu Ala Leu Pro Ala Leu Leu
   290 295
<210> 13
   <211> 13
   <212> PRT
   <213> Homo Sapiens
<400> 13
   Gln Leu Tyr Glu Asn Lys Pro Arg Arg Pro Tyr Ile Leu
   1 5 10
<210> 14
   <211> 3
   <212> PRT
   <213> Homo Sapiens
<400> 14
   Tyr Ile Leu
   1
<210> 15
   <211> 4
   <212> PRT
   <213> Homo Sapiens
<400> 15
   Pro Tyr Ile Leu
   1
<210> 16
   <211> 211
   <212> PRT
   <213> Homo Sapiens
<400> 16
   Met Lys Leu Trp Asp Val Val Ala Val Cys Leu Val Leu Leu His Thr
   1               5                   10                  15
   Ala Ser Ala Phe Pro Leu Pro Ala Gly Lys Arg Pro Pro Glu Ala Pro
               20                  25                  30
   Ala Glu Asp Arg Ser Leu Gly Arg Arg Arg Ala Pro Phe Ala Leu Ser
           35                  40                  45
   Ser Asp Ser Asn Met Pro Glu Asp Tyr Pro Asp Gln Phe Asp Asp Val
       50                  55                  60
   Met Asp Phe Ile Gln Ala Thr Ile Lys Arg Leu Lys Arg Ser Pro Asp
   65                  70                  75                  80
   Lys Gln Met Ala Val Leu Pro Arg Arg Glu Arg Asn Arg Gln Ala Ala
                   85                  90                  95
   Ala Ala Asn Pro Glu Asn Ser Arg Gly Lys Gly Arg Arg Gly Gln Arg
               100                 105                 110
   Gly Lys Asn Arg Gly Cys Val Leu Thr Ala Ile His Leu Asn Val Thr
           115                 120                 125
   Asp Leu Gly Leu Gly Tyr Glu Thr Lys Glu Glu Leu Ile Phe Arg Tyr
       130                 135                 140
   Cys Ser Gly Ser Cys Asp Ala Ala Glu Thr Thr Tyr Asp Lys Ile Leu
   145                 150                 155                 160
   Lys Asn Leu Ser Arg Asn Arg Arg Leu Val Ser Asp Lys Val Gly Gln
                   165                 170                 175
   Ala Cys Cys Arg Pro Ile Ala Phe Asp Asp Asp Leu Ser Phe Leu Asp
               180                 185                 190
   Asp Asn Leu Val Tyr His Ile Leu Arg Lys His Ser Ala Lys Arg Cys
           195                 200                 205
   Gly Cys Ile
       210
<210> 17
   <211> 58
   <212> PRT
   <213> Homo Sapiens
<400> 17
   Phe Pro Leu Pro Ala Gly Lys Arg Pro Pro Glu Ala Pro Ala Glu Asp
   1               5                   10                  15
   Arg Ser Leu Gly Arg Arg Arg Ala Pro Phe Ala Leu Ser Ser Asp Ser
               20                  25                  30
   Asn Met Pro Glu Asp Tyr Pro Asp Gln Phe Asp Asp Val Met Asp Phe
           35                  40                  45
   Ile Gln Ala Thr Ile Lys Arg Leu Lys Arg
       50                  55
<210> 18
   <211> 38
   <212> PRT
<213> Homo Sapiens
<400> 18
   Ser Pro Asp Lys Gln Met Ala Val Leu Pro Arg Arg Glu Arg Asn Arg
   1               5                   10                  15
   Gln Ala Ala Ala Ala Asn Pro Glu Asn Ser Arg Gly Lys Gly Arg Arg
               20                  25                  30
   Gly Gln Arg Gly Lys Asn
           35